(19) Europäisches Patentamt
European Patent Office
Office européen des brevets

(11) EP 4 340 056 A1

(12) EUROPEAN PATENT APPLICATION
published in accordance with Art. 153(4) EPC

(43) Date of publication:
**20.03.2024 Bulletin 2024/12**

(21) Application number: **22798914.2**

(22) Date of filing: **27.04.2022**

(51) International Patent Classification (IPC):
***H01M 4/131*** *(2010.01)* ***H01M 4/1391*** *(2010.01)*
***H01M 4/525*** *(2010.01)* ***H01M 4/62*** *(2006.01)*

(52) Cooperative Patent Classification (CPC):
**H01M 4/131; H01M 4/1391; H01M 4/525;**
**H01M 4/62;** Y02E 60/10

(86) International application number:
**PCT/JP2022/019167**

(87) International publication number:
**WO 2022/234809 (10.11.2022 Gazette 2022/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.05.2021 JP 2021079027**

(71) Applicant: **Daikin Industries, Ltd.**
**Osaka-shi, Osaka 530-0001 (JP)**

(72) Inventors:
- **ICHINOSE, Yuma**
  **Osaka-shi, Osaka 530-0001 (JP)**
- **YAMAZAKI, Shigeaki**
  **Osaka-shi, Osaka 530-0001 (JP)**
- **HOSODA, Chihiro**
  **Osaka-shi, Osaka 530-0001 (JP)**
- **ARAI, Kanako**
  **Osaka-shi, Osaka 530-0001 (JP)**
- **SHIMOOKA, Toshiharu**
  **Osaka-shi, Osaka 530-0001 (JP)**

(74) Representative: **Hoffmann Eitle**
**Patent- und Rechtsanwälte PartmbB**
**Arabellastraße 30**
**81925 München (DE)**

# (54) POSITIVE ELECTRODE MIXTURE, POSITIVE ELECTRODE AND SECONDARY BATTERY

(57) The present disclosure provides a positive electrode mixture comprising: a polyvinylidene fluoride (A); a positive electrode active material (C) represented by the general formula (C): $Li_yNi_{1-x}M_xO_2$, wherein x is $0.01 \leq x \leq 0.7$, y is $0.9 \leq y \leq 2.0$, and M represents a metal atom (excluding Li and Ni); and an additive (D), wherein the polyvinylidene fluoride (A) is a combination of: (A2) a polymer containing vinylidene fluoride unit and a fluorinated monomer unit; and at least one selected from the group consisting of (A3) a polymer containing vinylidene fluoride unit and a polar group-containing monomer unit, and (A4) a polymer containing vinylidene fluoride unit, a fluorinated monomer unit, and a polar group-containing monomer unit.

EP 4 340 056 A1

## Description

TECHNICAL FIELD

The present disclosure relates to a positive electrode mixture, a positive electrode, and a secondary battery.

BACKGROUND ART

Positive electrode mixtures used for forming positive electrodes in secondary batteries are required to have a characteristic that the change in viscosity after preparation is small, from the viewpoint of productivity of the positive electrode. For example, Patent Document 1 proposes, as an electrode mixture in which the change in viscosity is small even after 24 hours have passed since the preparation of the mixture, an electrode mixture comprising a powder electrode material, a binder, and an organic solvent, wherein the binder contains a fluorine-containing polymer composed of a polymerized unit derived from vinylidene fluoride and a polymerized unit derived from tetrafluoroethylene, and a polyvinylidene fluoride, the fluorine-containing polymer contains 80.0 to 90.0 mol% of the polymerized unit derived from vinylidene fluoride based on all polymerized units, and the polyvinylidene fluoride has a number average molecular weight of 150,000 to 1,400,000.

RELATED ART

PATENT DOCUMENT

Patent Document 1: International Publication No. WO 2013/176093

SUMMARY OF THE INVENTION

PROBLEM TO BE SOLVED BY THE INVENTION

However, there is a demand for a positive electrode mixture whose viscosity is even more unlikely to change even in the case where a positive electrode active material containing Ni in a high proportion is used in order to increase the capacity and voltage of secondary batteries and even after a long time has passed since its preparation, and that can also provide batteries having a high residual capacity rate and a high recovery capacity rate after high-temperature storage.

An object of the present disclosure is to provide a positive electrode mixture whose viscosity is unlikely to be increased despite comprising a positive electrode active material containing Ni in a high proportion and even after a long time has passed since its preparation, and that can provide batteries having a high residual capacity rate and a high recovery capacity rate after high-temperature storage.

MEANS FOR SOLVING THE PROBLEM

According to the present disclosure, provided is a positive electrode mixture comprising:

a polyvinylidene fluoride (A);
a positive electrode active material (C) represented by the general formula (C) : $Li_yNi_{1-x}M_xO_2$,

wherein x is $0.01 \leq x \leq 0.7$, y is $0.9 \leq y \leq 2.0$, and M represents a metal atom (excluding Li and Ni); and

an additive (D),
wherein the polyvinylidene fluoride (A) is a combination of:
(A2) a polymer containing vinylidene fluoride unit and a fluorinated monomer unit (excluding vinylidene fluoride unit and tetrafluoroethylene unit); and
at least one selected from the group consisting of (A3) a polymer containing vinylidene fluoride unit and a polar group-containing monomer unit, and (A4) a polymer containing vinylidene fluoride unit, a fluorinated monomer unit (excluding vinylidene fluoride unit and tetrafluoroethylene unit), and a polar group-containing monomer unit, and
wherein the additive (D) is at least one selected from the group consisting of: a diketone compound (1) represented by the general formula (1); an amine compound (2) represented by the general formula (2); an organophosphorus compound (3); a sulfur-containing compound (4); an aminocarbonate compound (5) represented by the general formula (5); a carboxylic acid (6) (excluding the aminocarbonate compound (5)); and an oxalato complex compound

(7):
the general formula (1):

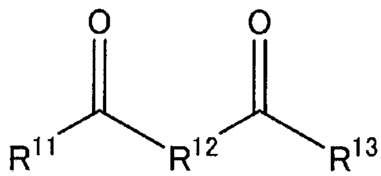

(1)

wherein $R^{11}$ and $R^{13}$ independently represent a monovalent hydrocarbon group optionally containing a heteroatom or a carbonyl group, $R^{12}$ represents a divalent hydrocarbon group or a divalent heteroatom, $R^{11}$, $R^{12}$, and $R^{13}$ are optionally bonded to each other to form a ring;
the general formula (2):

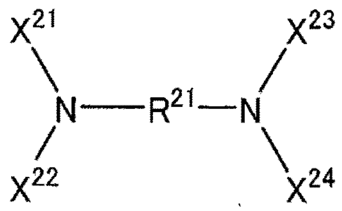

(2)

wherein $R^{21}$ represents a single bond or a divalent organic group, $X^{21}$ to $X^{24}$ independently represent a hydrogen atom, a substituted or unsubstituted alkyl group, or an aryl group optionally bonded to a nitrogen atom via a sulfonyl group, $X^{21}$ and $X^{23}$ are optionally bonded to each other to form a ring, and $X^{22}$ and $X^{24}$ are optionally bonded to each other to form a ring; and
the general formula (5):

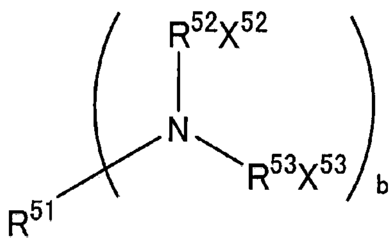

(5)

wherein $R^{51}$ represents an organic group, $R^{52}$ represents a single bond or an alkylene group, $X^{52}$ represents -OH or -$COOZ^{52}$, where $Z^{52}$ is a hydrogen atom or an alkali metal, $R^{53}$ represents a single bond or an alkylene group, $X^{53}$ is -H, -OH or -$COOZ^{53}$, where $Z^{53}$ is a hydrogen atom or an alkali metal, and b represents 1 or 2.

EFFECTS OF INVENTION

According to the present disclosure, it is possible to provide a positive electrode mixture whose viscosity is unlikely to be increased despite comprising a positive electrode active material containing Ni in a high proportion and even after a long time has passed since its preparation, and that can provide batteries having a high residual capacity rate and a high recovery capacity rate after high-temperature storage.

DESCRIPTION OF EMBODIMENTS

Hereinafter, specific embodiments of the present disclosure will be described in detail, but the present disclosure is not limited to the following embodiments.

A positive electrode mixture of the present disclosure comprises a polyvinylidene fluoride (A), a positive electrode active material (C), and an additive (D).

Polyvinylidene fluoride (A)

The polyvinylidene fluoride (PVdF) (A) is a combination of: (A2) a polymer containing vinylidene fluoride (VdF) unit and a fluorinated monomer unit (excluding VdF unit and tetrafluoroethylene (TFE) unit); and at least one selected from the group consisting of (A3) a polymer containing VdF unit and a polar group-containing monomer unit, and (A4) a polymer containing VdF unit, a fluorinated monomer unit (excluding VdF unit and TFE unit), and a polar group-containing monomer unit.

The content of VdF unit in the PVdF (A) is, since batteries having a higher residual capacity rate and a higher recovery capacity rate after high-temperature storage can be obtained, preferably 84.0 to 99.999 mol%, more preferably 90.0 mol% or more, still more preferably 92.0 mol% or more, yet more preferably 94.0 mol% or more, particularly preferably 95.0 mol% or more, and most preferably 97.0 mol% or more, based on all monomer units. The upper limit thereof is, in the order of preference, 99.99 mol% or less, 99.90 mol% or less, 99.899 mol% or less, 99.70 mol% or less, 99.50 mol% or less, 99.49 mol% or less, or 99.20 mol% or less.

In the present specification, the compositional features of the PVdF (A) can be measured by, for example, $^{19}F$-NMR measurement.

The fluorinated monomer unit described above contained in the PVdF (A2) and the PVdF (A4) is a fluorinated monomer unit that is different from VdF unit and TFE unit, and is preferably a monomer unit that has no polar group. Examples of the fluorinated monomer described above include vinyl fluoride, trifluoroethylene, chlorotrifluoroethylene (CTFE), a fluoroalkyl vinyl ether, hexafluoropropylene (HFP), a (perfluoroalkyl)ethylene, 2,3,3,3-tetrafluoropropene, and trans-1,3,3,3-tetrafluoropropene. Among these, at least one selected from the group consisting of CTFE, a fluoroalkyl vinyl ether, HFP, and 2,3,3,3-tetrafluoropropene is preferred, and at least one selected from the group consisting of HFP and a fluoroalkyl vinyl ether is more preferred. The fluorinated monomer unit described above contained in the PVdF (A2) and the PVdF (A4) may be the same or may be different.

Examples of the fluoroalkyl vinyl ether include a perfluoro(alkyl vinyl ether) (PAVE) represented by $CF_2$=CF-$ORf^1$ (wherein $Rf^1$ is a perfluoroalkyl group having 1 to 8 carbon atoms) and an alkyl perfluorovinyl ether derivative represented by $CF_2$=CF-$OCH_2$-$Rf^2$ (wherein $Rf^2$ is a perfluoroalkyl group having 1 to 5 carbon atoms). Among these, a PAVE is preferred, and at least one selected from the group consisting of perfluoro(methyl vinyl ether) (PMVE), perfluoro(ethyl vinyl ether) (PEVE), perfluoro(propyl vinyl ether) (PPVE), and perfluoro(butyl vinyl ether) is more preferred.

The content of the fluorinated monomer unit described above in the PVdF (A2) and the PVdF (A4) is preferably 0.10 to 10.0 mol%, more preferably 0.30 to 8.0 mol%, and still more preferably 0.50 to 6.0 mol%, based on all monomer units.

The PVdF (A3) and the PVdF (A4) have a polar group contained in the polar group-containing monomer unit described above, and as a result of this, batteries having a high residual capacity rate and a high recovery capacity rate after high-temperature storage can be obtained. The polar group described above is not limited as long as it is a functional group having polarity, but since batteries having a higher residual capacity rate and a higher recovery capacity rate after high-temperature storage can be obtained, it is preferably at least one selected from the group consisting of a carbonyl group-containing group, an epoxy group, a hydroxy group, a sulfonic acid group, a sulfuric acid group, a phosphoric acid group, an amino group, an amide group, and an alkoxy group, more preferably at least one selected from the group consisting of a carbonyl group-containing group, an epoxy group, and a hydroxy group, and still more preferably a carbonyl group-containing group. The hydroxy group described above does not include a hydroxy group that constitutes part of the carbonyl group-containing group described above. Also, the amino group described above is a monovalent functional group obtained by removing hydrogen from ammonia, or a primary or secondary amine.

The carbonyl group-containing group described above is a functional group having a carbonyl group (-C(=O)-). The carbonyl group-containing group described above is, since batteries having a higher residual capacity rate and a higher recovery capacity rate after high-temperature storage can be obtained, preferably a group represented by the general formula: -COOR (R represents a hydrogen atom, an alkyl group, or a hydroxyalkyl group) or a carboxylic anhydride group, and more preferably a group represented by the general formula: -COOR. The number of carbon atoms in the alkyl group and the hydroxyalkyl group is preferably 1 to 16, more preferably 1 to 6, and still more preferably 1 to 3. Specific examples of the group represented by the general formula: -COOR include - $COOCH_2CH_2OH$, -$COOCH_2CH(CH_3)OH$, -$COOCH(CH_3)CH_2OH$, -COOH, -$COOCH_3$, and -$COOC_2H_5$. In the case where the group represented by the general formula: -COOR is - COOH or contains -COOH, -COOH may be a carboxylic acid salt, such as a carboxylic acid metal salt or a carboxylic acid ammonium salt.

The amide group described above is preferably a group represented by the general formula: -CO-NRR' (R and R' independently represent a hydrogen atom or a substituted or unsubstituted alkyl group) or a bond represented by the general formula: -CO-NR''- (R'' represents a hydrogen atom, a substituted or unsubstituted alkyl group, or a substituted or unsubstituted phenyl group).

In the present specification, in the case where the polar group is an acid group, such as a carboxylic acid, the content of the polar group-containing monomer unit in the PVdF (A) can be measured by acid-base titration of the acid

group.

Examples of the polar group-containing monomer unit described above contained in the PVdF (A3) and the PVdF (A4) include a hydroxyalkyl (meth)acrylate such as hydroxyethyl acrylate and 2-hydroxypropyl acrylate; (meth)acrylic acid; an alkylidene malonic acid ester such as dimethyl methylidene malonate; a vinyl carboxyalkyl ether such as vinyl carboxymethyl ether and vinyl carboxyethyl ether; a carboxyalkyl (meth)acrylate such as 2-carboxyethyl acrylate and 2-carboxyethyl methacrylate; a (meth)acryloyloxyalkyl dicarboxylic acid ester such as acryloyloxyethyl succinate, methacryloyloxyethyl succinate, acryloyloxyethyl phthalate, and methacryloyloxyethyl phthalate; and a monoester of an unsaturated dibasic acid such as maleic acid monomethyl ester, maleic acid monoethyl ester, citraconic acid monomethyl ester, and citraconic acid monoethyl ester. Note that, in the present disclosure, "(meth)acrylic acid" means either acrylic acid or methacrylic acid, and may be a mixture of acrylic acid and methacrylic acid. The " (meta) " in other compound names is interpreted in the same manner.

As for the polar group-containing monomer contained in the PVdF (A3) and the PVdF (A4), among these, since batteries having a higher residual capacity rate and a higher recovery capacity rate after high-temperature storage can be obtained, at least one selected from the group consisting of a hydroxyalkyl (meth)acrylate, (meth)acrylic acid, an alkylidene malonic acid ester, a vinyl carboxyalkyl ether, a carboxyalkyl (meth)acrylate, a (meth)acryloyloxyalkyl dicarboxylic acid ester, and a monoester of an unsaturated dibasic acid is preferred, and at least one selected from the group consisting of (meth)acrylic acid, an alkylidene malonic acid ester, a (meth)acryloyloxyalkyl dicarboxylic acid ester, and a monoester of an unsaturated dibasic acid is more preferred.

The content of the polar group-containing monomer unit described above in the PVdF (A3) and the PVdF (A4) is preferably 0.001 to 8.0 mol%, more preferably 0.01 to 5.0 mol%, and still more preferably 0.30 to 3.0 mol%, based on all monomer units.

In the PVdF (A2), the content ratio of VdF unit and the fluorinated monomer unit is preferably (92.00 to 99.90)/(0.10 to 8.00), and more preferably (94.00 to 99.50)/(0.50 to 6.00), in molar ratio.

In the PVdF (A3), the content ratio of VdF unit and the polar group-containing monomer unit described above is preferably (92.00 to 99.999)/(0.001 to 8.00), more preferably (95.00 to 99.99)/(0.01 to 5.00), and still more preferably (97.00 to 99.70)/(0.30 to 3.00), in molar ratio.

In the PVdF (A4), the content ratio of VdF unit, the fluorinated monomer unit described above, and the polar group-containing monomer unit described above is preferably (84.00 to 99.899)/(0.10 to 8.00)/(0.001 to 8.00), more preferably (90.00 to 99.49)/(0.50 to 5.00)/(0.01 to 5.00), and still more preferably (92.00 to 99.20)/(0.50 to 5.00)/(0.30 to 3.00), in molar ratio.

The PVdF (A2), the PVdF (A3), and the PVdF (A4) may contain a non-fluorinated monomer unit. Examples of the non-fluorinated monomer described above include ethylene and propylene.

The weight average molecular weight (in terms of polystyrene) of the PVdF (A) is preferably 200,000 to 2,400,000, more preferably 400,000 or more, and still more preferably 600,000 or more, and it is more preferably 2,200,000 or less, and still more preferably 2,000,000 or less. The weight average molecular weight described above can be measured by gel permeation chromatography (GPC) using dimethylformamide as the solvent.

The number average molecular weight (in terms of polystyrene) of the PVdF (A) is preferably 70,000 to 1,200,000, and more preferably 140,000 or more, and it is more preferably 1,100,000 or less. The number average molecular weight described above can be measured by gel permeation chromatography (GPC) using dimethylformamide as the solvent.

The PVdF (A) preferably has a melting point of 100 to 240°C. The melting point described above can be determined as the temperature corresponding to the local maximum value in the heat-of-fusion curve when the temperature is increased at a rate of 10°C/min using a differential scanning calorimetry (DSC) apparatus.

The PVdF (A) can be produced by, for example, a conventionally known method such as mixing VdF and the polar group-containing monomer described above, as well as an additive such as a polymerization initiator, as appropriate, and carrying out solution polymerization or suspension polymerization.

In the positive electrode mixture of the present disclosure, the mass ratio between the PVdF (A2) and at least one selected from the group consisting of the PVdF (A3) and the PVdF (A4) is, since batteries having a higher residual capacity rate and a higher recovery capacity rate after high-temperature storage can be obtained, preferably 95/5 to 5/95, more preferably 90/10 to 10/90, still more preferably 85/15 to 15/85, and particularly preferably 80/20 to 20/80.

In the positive electrode mixture of the present disclosure, the content of the PVdF (A) is preferably 20% by mass or less, more preferably 10% by mass or less, and still more preferably 5% by mass or less, and it is preferably 0.1% by mass or more, and more preferably 0.5% by mass or more, based on the positive electrode mixture described above. When the content of the PVdF (A) is in the range described above, batteries having a higher residual capacity rate and a higher recovery capacity rate after high-temperature storage can be obtained.

The positive electrode mixture of the present disclosure may comprise another polymer in addition to the PVdF (A). Examples of the other polymer include a polymethacrylate, a polymethyl methacrylate, a polyacrylonitrile, a polyimide, a polyamide, a polyamide-imide, a polycarbonate, a styrene rubber, and a butadiene rubber.

Positive electrode active material (C)

The positive electrode active material (C) is represented by the general formula (C) : $Li_yNi_{1-x}M_xO_2$, wherein x is $0.01 \leq x \leq 0.7$, y is $0.9 \leq y \leq 2.0$, and M represents a metal atom (excluding Li and Ni). Since the positive electrode mixture of the present disclosure comprises a positive electrode mixture containing Ni in such a high amount, it is beneficial to increase the capacity of secondary batteries. In the prior art, when a positive electrode mixture containing a large amount of Ni was used, it was not possible to sufficiently suppress an increase in the viscosity of the positive electrode mixture, but in the present disclosure, an increase in the viscosity of the positive electrode mixture can be sufficiently suppressed, thus enabling achievement of both increased capacity of secondary batteries and improved productivity of secondary batteries.

In the general formula (C), examples of the metal atom of M include V, Ti, Cr, Mn, Fe, Co, Cu, Al, Zn, Mg, Ga, Zr, and Si. As the metal atom of M, a transition metal such as V, Ti, Cr, Mn, Fe, Co, and Cu, or a combination of the transition metal described above and another metal such as Al, Ti, V, Cr, Mn, Fe, Co, Cu, Zn, Mg, Ga, Zr, and Si is preferred.

Examples of the positive electrode active material (C) include a lithium transition metal composite oxide. At least one selected from the group consisting of $LiNi_{0.80}Co_{0.15}Al_{0.05}O_2$, $LiNi_{0.82}Co_{0.15}Al_{0.03}O_2$, $LiNi_{0.33}Mn_{0.33}Co_{0.33}O_2$, $LiNi_{0.5}Mn_{0.3}Co_{0.2}O_2$, $LiNi_{0.6}Mn_{0.2}Co_{0.2}O_2$, $LiNi_{0.8}Mn_{0.1}Co_{0.1}O_2$, and $LiNi_{0.90}Mh_{0.05}Co_{0.05}O_2$ is preferred, and at least one selected from the group consisting of $LiNi_{0.82}Co_{0.15}Al_{0.03}O_2$, $LiNi_{0.6}Mh_{0.2}Co_{0.2}O_2$, and $LiNi_{0.8}Mh_{0.1}Co_{0.1}O_2$ is more preferred.

In addition to the positive electrode active material (C), a different positive electrode active material may be used in combination. Specific examples of the different positive electrode active material include $LiNiO_2$, $LiCoO_2$, $LiMnO_2$, $LiMn_2O_4$, $Li_2MnO_3$, $LiMn_{1.8}Al_{0.2}O_4$, $Li_4Ti_5O_{12}$, $LiFePO_4$, $Li_3Fe_2(PO_4)_3$, $LiFeP_2O_7$, $LiCoPO_4$, and $Li_{1.2}Fe_{0.4}Mn_{0.4}O_2$.

It is also possible to use, as the positive electrode active material (C), a positive electrode active material (C) on the surface of which a substance with compositional features different from those of the substance constituting the main positive electrode active material (C) is attached. Examples of the surface-attached substance include an oxide such as aluminum oxide, silicon oxide, titanium oxide, zirconium oxide, magnesium oxide, calcium oxide, boron oxide, antimony oxide, and bismuth oxide; a sulfuric acid salt such as lithium sulfate, sodium sulfate, potassium sulfate, magnesium sulfate, calcium sulfate, and aluminum sulfate; and a carbonic acid salt such as lithium carbonate, calcium carbonate, and magnesium carbonate.

These surface-attached substances can be attached to the positive electrode active material (C) surface by, for example, a method in which they are dissolved or suspended in a solvent to impregnate and add to the positive electrode active material (C) and then dried, a method in which precursors of the surface-attached substances are dissolved or suspended in a solvent to impregnate and add to the positive electrode active material (C) and then allowed to react by heating or other means, and a method in which they are added to a precursor of the positive electrode active material and sintered at the same time.

The surface-attached substance is used in an amount of preferably 0.1 ppm or more, more preferably 1 ppm or more, and still more preferably 10 ppm or more, and preferably 20% or less, more preferably 10% or less, and still more preferably 5% or less based on the mass of the positive electrode active material (C). By the surface-attached substance, the oxidation reaction of the non-aqueous electrolyte on the surface of the positive electrode active material (C) can be suppressed and the battery life can be improved. However, in the case where the amount of the substance attached is too small, the effect is not fully expressed, and in the case where the amount is too large, the lithium ions may be inhibited from entering and leaving, resulting in an increased resistance.

The shape of particles of the positive electrode active material (C) used can be lumpy, polyhedral, spherical, ellipsoidal, plate-like, needle-like, columnar, or other shapes as conventionally used, but one in which primary particles are aggregated to form secondary particles and the shape of the secondary particles is spherical or ellipsoidal is preferred. Usually, as electrochemical elements are charged and discharged, the active material in the electrode expands and contracts, and this stress is likely to cause deterioration such as destruction of the active material and breakage of the conductive path. Therefore, rather than a single particle active material composed only of primary particles, one in which primary particles are aggregated to form secondary particles is preferred since the stress of expansion and contraction is relieved and deterioration is prevented. In addition, rather than axially oriented particles such as plate-like particles, spherical or ellipsoidal particles are preferred since they are less oriented at the time of electrode formation, resulting in less expansion and contraction of the electrode at the time of charging and discharging, and also easier to be mixed uniformly when mixed with the conductive agent at the time of preparing the electrode.

The tap density of the positive electrode active material (C) is usually 1.3 g/cm$^3$ or more, preferably 1.5 g/cm$^3$ or more, still more preferably 1.6 g/cm$^3$ or more, and most preferably 1.7 g/cm$^3$ or more. When the tap density of the positive electrode active material (C) is below the lower limit described above, while the amount of the dispersion medium required at the time of forming the positive electrode active material (C) layer is increased, the amounts of the conductive agent, and the PVdF (A) required are also increased, which may restrict the filling ratio of the positive electrode active material (C) in the positive electrode mixture layer, thereby restricting the battery capacity. By using a positive electrode

active material (C) with a high tap density, a positive electrode mixture layer with a high density can be formed. Although a larger tap density is preferred in general and there is no particular upper limit, if it is too large, the diffusion of lithium ions in the positive electrode mixture layer using the non-aqueous electrolyte as the medium may be rate-limiting, causing the load characteristic to be easily degraded. Therefore, it is usually 2.5 $g/cm^3$ or less, and preferably 2.4 $g/cm^3$ or less.

The tap density of the positive electrode active material (C) is obtained as follows. The sample is dropped through a sieve having an opening size of 300 μm into a 20-$cm^3$ tapping cell to fill the cell volume, tapping is then carried out 1,000 times over a stroke length of 10 mm using a powder densimeter (for example, Tap Denser manufactured by Seishin Enterprise Co., Ltd.), and the density determined from the volume and the weight of the sample at that time is defined as the tap density.

The median diameter d50 of particles of the positive electrode active material (C) (secondary particle size in the case where primary particles are aggregated to form secondary particles) is usually 0.1 μm or more, preferably 0.5 μm or more, more preferably 1 μm or more, and most preferably 3 μm or more, and it is usually 20 μm or less, preferably 18 μm or less, more preferably 16 μm or less, and most preferably 15 μm or less. When it is below the lower limit described above, products with a high bulk density may not be obtained, and when it exceeds the upper limit, the diffusion of lithium in the particles takes time, resulting in degraded battery performance, or problems such as streaking may occur when preparing a battery positive electrode, that is, slurrying the positive electrode active material (C), conductive agent, PVdF (A), and other materials with a solvent and applying them in the form of a thin film. Here, by mixing two or more types of positive electrode active materials (C) with different median diameters d50, the filling ability at the time of preparing the positive electrode can also be further improved.

Note that the median diameter d50 in the present disclosure is measured by a known laser diffraction and scattering type particle size distribution measuring apparatus. In the case of using LA-920 manufactured by HORIBA, Ltd. as a particle size analyzer, the measurement is performed using a 0.1 mass% sodium hexametaphosphate aqueous solution as the dispersion medium used in the measurement, and setting the measurement refractive index to 1.24 after ultrasonic dispersion for 5 minutes.

In the case where primary particles are aggregated to form secondary particles, the average primary particle size of the positive electrode active material (C) is usually 0.01 μm or more, preferably 0.05 μm or more, still more preferably 0.08 μm or more, and most preferably 0.1 μm or more, and it is usually 3 μm or less, preferably 2 μm or less, still more preferably 1 μm or less, and most preferably 0.6 μm or less. When it exceeds the upper limit described above, it may be difficult to form spherical secondary particles, which may adversely affect the powder filling ability or significantly decrease the specific surface area. Therefore, there may be a higher possibility of battery performance, such as power output characteristic, being degraded. On the other hand, when the average primary particle size is below the lower limit described above, crystals are usually underdeveloped, resulting in problems such as poor reversibility of charging and discharging. Note that the primary particle size is measured by observation using a scanning electron microscope (SEM). Specifically, it is determined by finding the longest value of the intercept by the left and right boundaries of a primary particle relative to a horizontal straight line in a photograph at a magnification of 10,000 times, for arbitrary 50 primary particles, and then averaging the values.

The BET specific surface area of the positive electrode active material (C) is usually 0.2 $m^2/g$ or more, preferably 0.3 $m^2/g$ or more, and still more preferably 0.4 $m^2/g$ or more, and it is usually 4.0 $m^2/g$ or less, preferably 2.5 $m^2/g$ or less, and still more preferably 1.5 $m^2/g$ or less. When the BET specific surface area is smaller than this range, battery performance is likely to be degraded, and when it is larger, the tap density is unlikely to rise, and problems may easily occur in the applicability of the positive electrode mixture.

The BET specific surface area is obtained as follows. Using a surface area analyzer (for example, a fully automatic surface area measuring apparatus manufactured by Ohkura Riken Co., Ltd.), the sample is pre-dried at 150°C for 30 minutes under nitrogen circulation, and then using a nitrogen-helium mixed gas precisely adjusted so that the relative pressure of nitrogen to atmospheric pressure is 0.3, measurement is performed by the single point BET method according to the gas flow method and the obtained value is defined as the BET specific surface area.

As the method for producing the positive electrode active material (C), a general method for producing inorganic compounds is used. In particular, a variety of methods can be considered in order to prepare spherical or ellipsoidal active materials, and examples thereof include: a method in which a transition metal raw material substance such as a transition metal nitrate or sulfate, as well as a raw material substance of another element if necessary, is dissolved or pulverized and dispersed in a solvent such as water, the pH is adjusted while stirring to prepare and collect a spherical precursor, which is dried as necessary, and then a Li source such as LiOH, $Li_2CO_3$, or $LiNO_3$ is added and sintered at a high temperature to obtain an active material; a method in which a transition metal raw material substance such as a transition metal nitrate, sulfate, hydroxide, or oxide, as well as a raw material substance of another element if necessary, is dissolved or pulverized and dispersed in a solvent such as water, dried and formed into a spherical or ellipsoidal precursor using a spray dryer or other means, and a Li source such as LiOH, $Li_2CO_3$, or $LiNO_3$ is added to this and sintered at a high temperature to obtain an active material; and a method in which a transition metal raw material substance such as a transition metal nitrate, sulfate, hydroxide, or oxide and a Li source such as LiOH, $Li_2CO_3$, or $LiNO_3$,

as well as a raw material substance of another element if necessary, are dissolved or pulverized and dispersed in a solvent such as water, dried and formed into a spherical or ellipsoidal precursor using a spray dryer or other means, and then sintered at a high temperature to obtain an active material.

Note that one type of positive electrode active material (C) may be used alone, or two or more types thereof with different compositional features or different powder physical properties may be used together in an arbitrary combination and ratio.

In the positive electrode mixture of the present disclosure, the mass ratio between the PVdF (A) and the positive electrode active material (C) is, since batteries having an even higher residual capacity rate and an even higher recovery capacity rate after high-temperature storage can be obtained, preferably 0.01/99.99 to 10/90, more preferably 0.5/99.5 to 4/96, and still more preferably 1/99 to 3/97.

Additive (D)

The positive electrode mixture of the present disclosure comprises at least one additive (D) selected from the group consisting of: a diketone compound (1); an amine compound (2); an organophosphorus compound (3); a sulfur-containing compound (4); an aminocarbonate compound (5); a carboxylic acid (6); and an oxalato complex compound (7).

Diketone compound (1)

The diketone compound (1) is represented by the general formula (1):
the general formula (1):

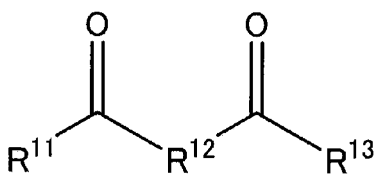

(1)

wherein $R^{11}$ and $R^{13}$ independently represent a monovalent hydrocarbon group optionally containing a heteroatom or a carbonyl group, $R^{12}$ represents a divalent hydrocarbon group or a divalent heteroatom, $R^{11}$, $R^{12}$, and $R^{13}$ are optionally bonded to each other to form a ring.

In the general formula (1), an oxygen atom, a sulfur atom, or a halogen atom is preferred as the heteroatom optionally contained in the hydrocarbon group of $R^{11}$ and $R^{13}$. An oxygen atom is preferred as the divalent heteroatom optionally contained in $R^{12}$.

In the general formula (1), as $R^{11}$ and $R^{13}$, independently, a linear or branched non-fluorinated alkyl group having 1 to 6 carbon atoms, a linear or branched fluorinated alkyl group having 1 to 6 carbon atoms, an aryl group, a thienyl group, a furyl group, a vinylene group formed by $R^{11}$ and $R^{13}$ bonded to each other, or a linear or branched non-fluorinated alkylene group having 2 to 6 carbon atoms formed by $R^{11}$ and $R^{13}$ bonded to each other is preferred, and a linear or branched non-fluorinated alkyl group having 1 to 6 carbon atoms or a linear or branched fluorinated alkyl group having 1 to 6 carbon atoms is more preferred.

In the general formula (1), $R^{12}$ is preferably a linear or branched alkylene group having 1 to 4 carbon atoms or an oxygen atom.

Alternatively, in the general formula (1), $R^{11}$ and $R^{12}$ are optionally bonded to each other to form a ring. As such a ring, an aliphatic ring having 3 to 7 carbon atoms formed by $R^{11}$ and $R^{12}$, together with the carbonyl group to which they are bonded, or a substituted or unsubstituted oxygen-containing heterocyclic ring formed together with the carbonyl group to which they are bonded and further containing another carbonyl group is preferred.

Examples of the diketone compound (1) include acetylacetone, 2-acetylcyclopentanone, 2-acetylcyclohexanone, 1,3-bis(4-methoxyphenyl)-1,3-propanedione, 3-methyl-2,4-pentanedione, 1-(4-tert-butylphenyl)-3-(4-methoxyphenyl)-1,3-propanedione, 1-(4-bromophenyl)-1,3-butanedione, 3-chloroacetylacetone, 6-methyl-2,4-heptanedione, 1-(4-chlorophenyl)-4,4,4-trifluoro-1,3-butanedione, dehydroacetic acid, 1,3-diphenyl-1,3-propanedione, 3,5-heptanedione, dipivaloylmethane, 2,2-dimethyl-6,6,7,7,8,8,8-heptafluoro-3,5-octanedione, 2,6-dimethyl-3,5-heptanedione, 4,4,4-trifluoro-1-(2-thienyl)-1,3-butanedione, hexafluoroacetylacetone, trifluoroacetylacetone, 1-(4-fluorophenyl)-4,4,4-trifluoro-1,3-butanedione, 2-furoyltrifluoroacetone, maleic anhydride, and succinic anhydride.

Among these, since an increase in the viscosity of the positive electrode mixture can be further suppressed, at least one selected from the group consisting of 3-methyl-2,4-pentanedione, acetylacetone, 3,5-heptanedione, hexafluoroacetylacetone, and trifluoroacetylacetone is preferred as the diketone compound (1).

Amine compound (2)

The amine compound (2) is represented by the general formula (2):
the general formula (2):

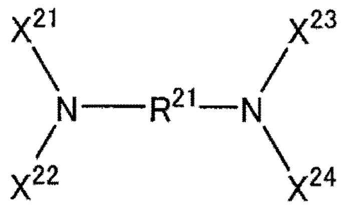

(2)

wherein $R^{21}$ represents a single bond or a divalent organic group; $X^{21}$ to $X^{24}$ independently represent a hydrogen atom, a substituted or unsubstituted alkyl group, a substituted or unsubstituted aminoalkyl group, or an aryl group optionally bonded to a nitrogen atom via a sulfonyl group; $X^{21}$ and $X^{23}$ are optionally bonded to each other to form a ring, and $X^{22}$ and $X^{24}$ are optionally bonded to each other to form a ring.

In the general formula (2), as the divalent organic group, a linear or branched alkylene group having 1 to 4 carbon atoms or a cycloalkylene group having 5 to 8 carbon atoms, in which a part of hydrogen atoms is optionally substituted with an aryl group, is preferred.

In the general formula (2), as the alkyl group of $X^{21}$ to $X^{24}$, a linear or branched alkyl group having 1 to 8 carbon atoms is preferred. As the aminoalkyl group of $X^{21}$ to $X^{24}$, a linear or branched aminoalkyl group having 1 to 8 carbon atoms is preferred. As the aryl group of $X^{21}$ to $X^{24}$, a hydroxyphenyl group, a methyl phenyl group, or a trimethyl phenyl group, optionally bonded to a nitrogen atom via a sulfonyl group is preferred.

As $X^{21}$ to $X^{24}$ in the general formula (2), a hydrogen atom or a substituted or unsubstituted aminoalkyl group is preferred, and a hydrogen atom or an aminoalkyl group having 1 to 8 carbon atoms is more preferred.

Alternatively, in the general formula (2), $X^{21}$ and $X^{23}$ are optionally bonded to each other to form a ring, and $X^{22}$ and $X^{24}$ are optionally bonded to each other to form a ring. As such a ring, a nitrogen-containing heterocyclic ring formed together with the two nitrogen atoms to which they are bonded and $R^{21}$ is preferred.

Examples of the amine compound (2) include (R,R)-N-(2-amino-1,2-diphenylethyl)-p-toluenesulfonamide, (S,S)-N-(2-amino-1,2-diphenylethyl)-p-toluenesulfonamide, (1R,2R)-1,2-bis(2,4,6-trimethylphenyl)ethylenediamine, (1S,2S)-1,2-bis(2,4,6-trimethylphenyl)ethylenediamine, (1R,2R)-1,2-bis(2-hydroxyphenyl)ethylenediamine, (1S,2S)-1,2-bis(2-hydroxyphenyl)ethylenediamine, N,N',N''-trimethyldiethylenetriamine, (1R,2R)-(-)-1,2-cyclohexanediamine, (1S,2S)-(+)-1,2-cyclohexanediamine, 1,4-diazabicyclo[2.2.2]octane, diethylenetriamine, N,N'-dimethylethylenediamine, N,N'-diphenylethylenediamine, (1S,2S)-(-)-1,2-diphenylethylenediamine, (1R,2R)-(+)-1,2-diphenylethylenediamine, (R,R)-(-)-2,3-dimethoxy-1,4-bis(dimethylamino)butane, (S,S)-(+)-2,3-dimethoxy-1,4-bis(dimethylamino)butane, (1R,2R)-(-)-N,N'-dimethylcyclohexane-1,2-diamine, (1S,2S)-(+)-N,N'-dimethylcyclohexane-1,2-diamine, N,N'-di-tert-butylethylenediamine, (1R,2R)-N,N'-dimethyl-N,N'-bis(3,3-dimethylbutyl)cyclohexane-1,2-diamine, (1S,2S)-N,N'-dimethyl-N,N'-bis(3,3-dimethylbutyl)cyclohexane-1,2-diamine, ethylenediamine, 1,3-diaminopropane, 1,4-diaminobutane, and triethylenetetramine.

Among these, since an increase in the viscosity of the positive electrode mixture can be further suppressed, at least one selected from the group consisting of ethylenediamine, 1,3-diaminopropane, 1,4-diaminobutane, diethylenetriamine, and triethylenetetramine is preferred as the amine compound (2) .

Organophosphorus compound (3)

The organophosphorus compound (3) is a compound containing at least one carbon atom-phosphorus atom bond. As the organophosphorus compound (3), an organophosphorus compound (3) (excluding the oxalato complex compound (7)), is preferred, and a phosphine compound is more preferred.

Examples of the organophosphorus compound (3) include a compound represented by the general formula (3-1) or a general formula (3-2) :

the general formula (3-1):

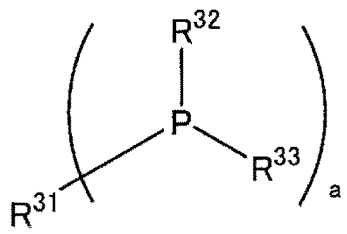

(3-1)

the general formula (3-2):

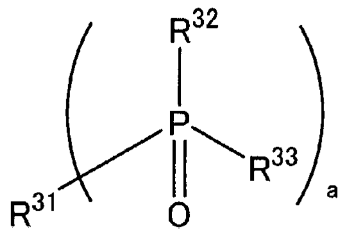

(3-2)

wherein a represents 1 or 2; $R^{31}$ represents a hydrogen atom, a linear, branched, or cyclic, substituted or unsubstituted hydrocarbon group, a substituted or unsubstituted metallocenyl group, or a substituted or unsubstituted heterocyclic group; $R^{32}$ and $R^{33}$ independently represent an alkyl group, a substituted or unsubstituted cycloalkyl group, a substituted or unsubstituted heterocyclic group, or an aryl group; and in the case where a is 2, there are two each of $R^{32}$ and $R^{33}$, which may be the same or different.

The compound represented by the general formula (3-1) or the general formula (3-2) can also form a salt together with an anion such as a halide anion or a tetrafluoroboric acid anion.

In the general formula (3-1) and the general formula (3-2), the hydrocarbon group can also contain a heteroatom such as an oxygen atom and a nitrogen atom. The number of carbon atoms in the hydrocarbon group described above may be 1 to 40.

In the general formula (3-1) and the general formula (3-2), the number of carbon atoms in the alkyl group may be 1 to 30. The number of carbon atoms in the cycloalkyl group may be 3 to 8. As the aryl group, a phenyl group optionally substituted with an alkyl group, an amino group, a halogen atom, a nitrile group, an alkoxy group, or a sulfo group is preferred.

Examples of the organophosphorus compound (3) having one phosphorus atom include 1,3-bis(2,6-diisopropylphenyl)-1,3,2-diazaphospholidine 2-oxide, (2-bromophenyl)diphenylphosphine, bis(pentafluorophenyl)phenylphosphine, 2-butenyl(di-tert-butyl)phosphine, cyclohexyldiphenylphosphine, di-tert-butyl(1-methyl-2,2-diphenylcyclopropyl)phosphine, dicyclohexyl(1-methyl-2,2-diphenylcyclopropyl)phosphine, dicyclohexyl(1,1-diphenyl-1-propen-2-yl)phosphine, diethylphenylphosphine, sodium diphenylphosphinobenzene-3-sulfonate, dicyclohexylphenylphosphine, methyl(diphenyl)phosphine oxide, diphenyl-2-pyridylphosphine, 4-(dimethylamino)phenyldiphenylphosphine, (R)-N,N-dimethyl-1-[(S)-2-(diphenylphosphino)ferrocenyl]ethylamine, (S)-N,N-dimethyl-1-[(R)-2-(diphenylphosphino)ferrocenyl]ethylamine, diphenylpropylphosphine, (R)-(+)-2-diphenylphosphino-2'-methoxy-1,1'-binaphthyl, 2-(diphenylphosphino)benzoic acid, 4-(diphenylphosphino)benzoic acid, (S)-1-(diphenylphosphino)-2-[(S)-4-isopropyloxazolin-2-yl]ferrocene, 1-[2-(di-tert-butylphosphino)phenyl]-3,5-diphenyl-1H-pyrazole, di-tert-butylphenylphosphine, 2-diphenylphosphino-2'-(N,N-dimethylamino)biphenyl, 2-(diphenylphosphino)benzaldehyde, (4-dimethylaminophenyl)di-tert-butylphosphine, di-tert-butyl(3-methyl-2-butenyl)phosphine, 2-(diphenylphosphino)biphenyl, di-1-adamantylphosphine, 3-(diphenylphosphino)-1-propylamine, 2-(diphenylphosphino)benzonitrile, 2-dicyclohexylphosphino-2'-methylbiphenyl, 2-dicyclohexylphosphino-2',6'-dimethoxybiphenyl, 2-dicyclohexylphosphino-2',6'-diisopropoxybiphenyl, 2-di-tert-butylphosphino-2',4',6'-triisopropylbiphenyl, dicyclohexyl(2,4,6-triisopropylphenyl)phosphine, ethyldiphenylphosphine, isopropyldiphenylphosphine, methoxymethyl(diphenyl)phosphine oxide, methyldiphenylphosphine, 1-methyl-1-[4-(diphenylphosphino)benzyl]pyrrolidinium bromide, (pentafluorophenyl)diphenylphosphine, 1,2,3,4,5-pentaphenyl-1'-(di-tert-butylphosphino)ferrocene, tributylphosphine, tri-n-octylphosphine, tri-n-octylphosphine oxide, triphenylphosphine, triphenylphosphine oxide, tris(4-methoxyphenyl)phosphine, tri(p-tolyl)phosphine, tributylphosphine oxide, trihexylphosphine, tri(o-tolyl)phosphine, tri(m-tolyl)phosphine, tricyclohexylphosphine, tris(2,6-dimethoxyphenyl)phosphine, tri(2-furyl)phosphine, tris(2-carboxyethyl)phosphine hydrochloride, tri(2-thienyl)phosphine, tri-tert-butylphosphine, triphenylphosphine-3,3',3"-trisulfonic acid trisodium salt, tricyclopentylphosphine, tris(pentafluorophenyl)phosphine, tris[3,5-bis(trifluoromethyl)phenyl]phosphine, tri-tert-butylphosphonium tetrafluoroborate, tricyclohexylphosphonium tetrafluoroborate, tris(4-fluorophenyl)phosphine, tris(2-methoxyphenyl)phosphine, trimethylphosphine, triethylphosphine, and di-tert-butyl(1,1-diphenyl-1-propen-2-yl)phosphine.

Examples of the organophosphorus compound (3) having two phosphorus atoms include (2S,3S)-(+)-1,4-bis(diphenylphosphino)-2,3-O-isopropylidene-2,3-butanediol, (2R,3R)-(-)-1,4-bis(diphenylphosphino)-2,3-O-isopropylidene-2,3-butanediol, bis(diphenylphosphino)methane, 1,2-bis(diphenylphosphino)ethane, 1,3-bis(diphenylphosphino)propane, 1,2-bis(dimethylphosphino)ethane, 1,4-bis(diphenylphosphino)butane, (S)-(-)-2,2'-bis(diphenylphosphino)-1,1'-binaphthyl, (R)-(+)-2,2'-bis(diphenylphosphino)-1,1'-binaphthyl, 1,6-bis(diphenylphosphino)hexane, 1,5-bis(diphenylphosphino)pentane, bis(diphenylphosphino)methane, trans-1,2-bis(diphenylphosphino)ethylene, 1,1'-bis(diphenylphosphino)ferrocene, (S,S)-1,2-bis[(tert-butyl)methylphosphino]ethane bis(borane), (±)-2,2'-bis(diphenylphosphino)-1,1'-binaphthyl, 2,2'-bis(diphenylphosphino)biphenyl, (2S,3S)-(-)-bis(diphenylphosphino)butane, 4,5-bis(diphenylphosphino)-9,9-dimethylxanthene, 1,1'-bis(diisopropylphosphino)ferrocene, 1,1'-bis(di-tert-butylphosphino)ferrocene, 4,6-bis(diphenylphosphino)phenoxazine, bis[2-(diphenylphosphino)phenyl] ether, (R,R)-1,2-bis[(2-methoxyphenyl)phenylphosphino]ethane, (S,S)-1,2-bis[(2-methoxyphenyl)phenylphosphino]ethane, (R,R")-2,2"-bis(diphenylphosphino)-1,1''-biferrocene, 1,2-bis(diphenylphosphino)benzene, 1,2-bis[bis(pentafluorophenyl)phosphino]ethane, (2R,3R)-(-)-2,3-bis(diphenylphosphino)bicyclo[2.2.1]hept-5-ene, (2S,3S)-(+)-2,3-bis(diphenylphosphino)bicyclo[2.2.1]hept-5-ene, 1,8-bis(diphenylphosphino)naphthalene, 1,3-bis[(di-tert-butylphosphino)oxy]benzene, 4,5-bis(dicyclohexylphosphino)-9,9-dimethylxanthene, (R,R)-(-)-2,3-bis(tert-butylmethylphosphino)quinoxaline, (S,S)-(+)-2,3-bis(tert-butylmethylphosphino)quinoxaline, N,N-bis[(diphenylphosphino)methyl]-3-(triethoxysilyl)propylamine, (R)-N,N-dimethyl-1-[(S)-1',2-bis(diphenylphosphino)ferrocenyl]ethylamine, and (S)-N,N-dimethyl-1-[(R)-1',2-bis(diphenylphosphino)ferrocenyl]ethylamine.

Also, as the organophosphorus compound (3), the compound represented by the general formula (3-1) is preferred. Furthermore, in the general formula (3-1), at least one selected from the group consisting of a compound wherein a is 1 and $R^{31}$ to $R^{33}$ are independently an alkyl group having 1 to 4 carbon atoms or a substituted or unsubstituted phenyl group, and a compound wherein a is 2, $R^{31}$ is a linear or branched alkylene group having 1 to 4 carbon atoms, and $R^{32}$ to $R^{33}$ are independently a substituted or unsubstituted phenyl group is more preferred.

As the organophosphorus compound (3), in particular, since an increase in the viscosity of the positive electrode mixture can be further suppressed, at least one selected from the group consisting of triphenylphosphine, trimethylphosphine, triethylphosphine, and a diphosphine compound represented by the general formula: $Ph_2$-P-$R^{31}$-P-$Ph_2$ (wherein $R^{31}$ represents a linear or branched alkylene group having 1 to 4 carbon atoms, and Ph represents a phenyl group) is still more preferred.

Sulfur-containing compound (4)

The sulfur-containing compound (4) is not limited as long as it is a compound containing a sulfur atom, but it is preferably an organosulfur compound and examples thereof include a sulfur-containing heterocyclic compound, a sulfonic acid ester compound, and a sulfuric acid ester compound. As the sulfur-containing compound (4), at least one selected from the group consisting of a sulfur-containing heterocyclic compound and a sulfonic acid ester compound is preferred, and a sulfur-containing heterocyclic compound is more preferred.

Examples of the sulfonic acid ester compound include ethyl methanesulfonate.

The sulfur-containing heterocyclic compound is a substituted or unsubstituted heterocyclic compound containing a sulfur atom, and may be a non-aromatic compound or an aromatic compound. Also, it may be a monocyclic compound, a polycyclic compound, or a fused-ring compound. The number of carbon atoms in the sulfur-containing heterocyclic compound is preferably 1 to 12.

Examples of the sulfur-containing heterocyclic compound include ethylene sulfide, trimethylene sulfide, tetrahydrothiophene, pentamethylene sulfide, thiophene, 2-thiophenecarboxylic acid, 2,5-dimethylthiophene, 2-methyltetrahydrothiophene, 2,2'-bithiophene, and 1,3-propanesultone.

As the sulfur-containing heterocyclic compound, at least one selected from the group consisting of a sulfur-containing heterocyclic saturated monocyclic compound having 2 to 6 carbon atoms, a sulfur-containing heterocyclic unsaturated monocyclic compound having 4 to 6 carbon atoms, and a sulfur-containing heterocyclic unsaturated polycyclic compound having 8 to 12 carbon atoms is preferred.

As the sulfur-containing heterocyclic compound, in particular, since an increase in the viscosity of the positive electrode mixture can be further suppressed, at least one selected from the group consisting of ethylene sulfide, trimethylene sulfide, tetrahydrothiophene, pentamethylene sulfide, thiophene, 2,2'-bithiophene, and 1,3-propanesultone is preferred.

Aminocarbonate compound (5)

The aminocarbonate compound (5) is an amine compound having a carboxy group, and it may be a hydroxyaminocarbonate compound in which a part or all of carboxy groups are replaced with a hydroxy group in the amine compound having a carboxy group described above. Also, the aminocarbonate compound (5) may be a hydrate.

The aminocarbonate compound (5) is represented by the general formula (5): the general formula (5):

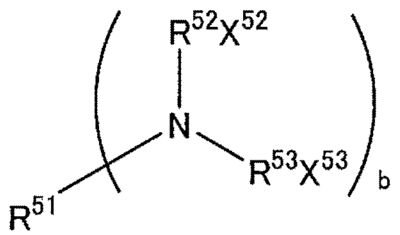

(5)

wherein $R^{51}$ represents an organic group, $R^{52}$ represents a single bond or an alkylene group, $X^{52}$ represents -OH or $-COOZ^{52}$, where $Z^{52}$ is a hydrogen atom or an alkali metal, $R^{53}$ represents a single bond or an alkylene group, $X^{53}$ is -H, -OH or $-COOZ^{53}$, where $Z^{53}$ is a hydrogen atom or an alkali metal, and b represents 1 or 2.

In the general formula (5), as the organic group, a linear or branched alkylene group having 1 to 4 carbon atoms, a substituted or unsubstituted cycloalkylene group having 4 to 6 carbon atoms, a substituted or unsubstituted alkylene group containing an oxygen atom, a nitrogen atom, or an arylene group, or $-R^{54}COOZ^{54}$ ($R^{54}$ is a single bond or an alkylene group, and $Z^{54}$ is a hydrogen atom or an alkali metal) is preferred.

Examples of the aminocarbonate compound (5) include 1,2-bis(2-aminophenoxy)ethane-N,N,N',N'-tetraacetic acid, trans-1,2-cyclohexanediaminetetraacetic acid monohydrate, diethylenetriaminepentaacetic acid (DTPA), 1,2-diaminopropane-N,N,N',N'-tetraacetic acid, 1,3-diamino-2-propanol-N,N,N',N'-tetraacetic acid, 1,6-diaminohexane-N,N,N',N'-tetraacetic acid, diammonium ethylenediaminetetraacetate monohydrate, disodium dihydrogen ethylenediaminetetraacetate dihydrate, dipotassium dihydrogen ethylenediaminetetraacetate dihydrate, ethylenediaminetetraacetic acid (EDTA), copper(II) disodium ethylenediaminetetraacetate tetrahydrate, magnesium disodium ethylenediaminetetraacetate hydrate, tetrasodium ethylenediaminetetraacetate dihydrate, ethylene glycol bis(2-aminoethyl ether)-N,N,N',N'-tetraacetic acid (GEDTA), ethylenediamine-N,N'-diacetic acid, ethylenediamine-N,N'-diacetic-N,N'-dipropionic acid hydrate, trisodium hydrogen ethylenediaminetetraacetate hydrate, N-(2-hydroxyethyl)ethylenediamine-N,N',N'-triacetic acid (HEDTA), nitrilotriacetic acid (NTA), 1,3-propanediamine-N,N,N',N'-tetraacetic acid, N,N,N',N'-tetrakis(2-hydroxyethyl)ethylenediamine, triethylenetetramine-N,N,N',N",N‴,N‴-hexaacetic acid (TTHA), tetrasodium N,N-bis(carboxymethyl)-L-glutamate (GLDA), hydroxyethyliminodiacetic acid (HIDA), and dihydroxyethylglycine (DHEG).

As the aminocarbonate compound (5), in particular, since an increase in the viscosity of the positive electrode mixture can be further suppressed, at least one selected from the group consisting of ethylenediaminetetraacetic acid (EDTA), nitrilotriacetic acid (NTA), diethylenetriaminepentaacetic acid (DTPA), tetrasodium N,N-bis(carboxymethyl)-L-glutamate (GLDA), N-(2-hydroxyethyl)ethylenediamine-N,N',N'-triacetic acid (HEDTA), ethylene glycol bis(2-aminoethyl ether)-N,N,N',N'-tetraacetic acid (GEDTA), triethylenetetramine-N,N,N' ,N' ',N''',N'''-hexaacetic acid (TTHA), hydroxyethyliminodiacetic acid (HIDA), and dihydroxyethylglycine (DHEG) is preferred.

Carboxylic acid (6)

The carboxylic acid (6) (excluding the aminocarbonate compound (5)), is an organic acid having $-COOZ^{64}$ ($Z^{64}$ is a hydrogen atom or an alkali metal) . It may be a monocarboxylic acid having one $-COOZ^{64}$ in one molecule, or may be a polycarboxylic acid having two or more $-COOZ^{64}$ in one molecule.

The number of carbon atoms in the carboxylic acid (6), including the number of carbon atoms constituting $-COOZ^{64}$, is preferably 1 to 12, and more preferably 1 to 6. The carboxylic acid (6) is preferably free from a nitrogen atom.

Examples of the carboxylic acid (6) include acetic acid, acrylic acid, formic acid, citric acid, oxalic acid, lactic acid, pyruvic acid, malonic acid, propionic acid, maleic acid, butyric acid, citraconic acid, tartaric acid, malic acid, and triglycolamic acid.

Among these, since an increase in the viscosity of the positive electrode mixture can be further suppressed, at least one selected from the group consisting of acetic acid, maleic acid, pyruvic acid, formic acid, malonic acid, acrylic acid, citraconic acid, tartaric acid, malic acid, and triglycolamic acid is preferred as the carboxylic acid (6).

Oxalato complex compound (7)

The oxalato complex compound (7) is preferably a complex compound formed by the coordination bond of at least one oxalate ion to a coordination atom.

Examples of the oxalato complex compound (7) include a compound represented by the general formula (7):

the general formula (7):

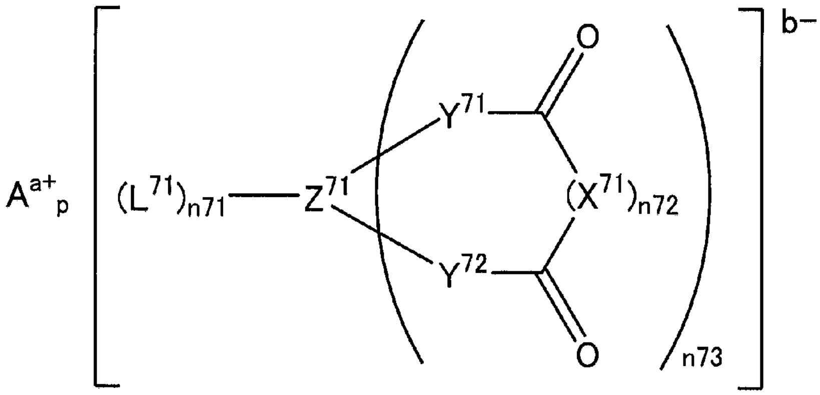

(7)

wherein $A^{a+}$ is a metal ion, a hydrogen ion, or an onium ion; a is an integer of 1 to 3, b is an integer of 1 to 3, p is b/a, n73 is an integer of 1 to 4, n71 is an integer of 0 to 8, n72 is 0 or 1, $Z^{71}$ is a transition metal or an element in Group III, Group IV, or Group V in the periodic table;

$X^{71}$ is O, S, an alkylene group having 1 to 10 carbon atoms, a halogenated alkylene group having 1 to 10 carbon atoms, an arylene group having 6 to 20 carbon atoms, or a halogenated arylene group having 6 to 20 carbon atoms (the alkylene group, the halogenated alkylene group, the arylene group, and the halogenated arylene group optionally have a substituent or a heteroatom in their structure, and when n72 is 1 and n73 is 2 to 4, n73 $X^{71}$ (2 to 4 $X^{71}$) are optionally bonded to each other) ;

$L^{71}$ is a halogen atom, a cyano group, an alkyl group having 1 to 10 carbon atoms, a halogenated alkyl group having 1 to 10 carbon atoms, an aryl group having 6 to 20 carbon atoms, a halogenated aryl group having 6 to 20 carbon atoms (the alkylene group, the halogenated alkylene group, the arylene group, and the halogenated arylene group optionally have a substituent or a heteroatom in their structure, and when n71 is 2 to 8, n71 $L^{71}$ (2 to 8 $L^{71}$) are optionally bonded to each other to form a ring), or $-Z^{73}Y^{73}$;

$Y^{71}$, $Y^{72}$, and $Z^{73}$ are each independently O, S, $NY^{74}$, a hydrocarbon group, or a fluorinated hydrocarbon group; and $Y^{73}$ and $Y^{74}$ are each independently H, F, an alkyl group having 1 to 10 carbon atoms, a halogenated alkyl group having 1 to 10 carbon atoms, an aryl group having 6 to 20 carbon atoms, or a halogenated aryl group having 6 to 20 carbon atoms (the alkyl group, the halogenated alkyl group, the aryl group, and the halogenated aryl group optionally have a substituent or a heteroatom in their structure, and in the case where there is a plurality of $Y^{73}$ or $Y^{74}$, they are optionally bonded to each other to form a ring).

Examples of $A^{a+}$ include a lithium ion, a sodium ion, a potassium ion, a magnesium ion, a calcium ion, a barium ion, a cesium ion, a silver ion, a zinc ion, a copper ion, a cobalt ion, an iron ion, a nickel ion, a manganese ion, a titanium ion, a lead ion, a chromium ion, a vanadium ion, a ruthenium ion, a yttrium ion, a lanthanoid ion, an actinoid ion, a tetrabutylammonium ion, a tetraethylammonium ion, a tetramethylammonium ion, a triethylmethylammonium ion, a triethylammonium ion, a pyridinium ion, an imidazolium ion, a hydrogen ion, a tetraethylphosphonium ion, a tetramethylphosphonium ion, a tetraphenylphosphonium ion, a triphenylsulfonium ion, and a triethylsulfonium ion.

When used in electrochemical devices and other applications, $A^{a+}$ is preferably a lithium ion, a sodium ion, a magnesium ion, a tetraalkylammonium ion, or a hydrogen ion, and particularly preferably a lithium ion. The valence a of the $A^{a+}$ cation is an integer of 1 to 3. In the case where the valence is larger than 3, the crystal lattice energy becomes larger, which causes a problem of difficulty in being dissolved in a solvent. Therefore, in the case where solubility is required, 1 is more preferred. The valence b of the anion is likewise an integer of 1 to 3, and 1 is particularly preferred. The constant p, which represents the ratio of the cation to the anion, is necessarily determined by the ratio of their valences, b/a.

Next, the ligand moiety of the general formula (7) will be described. In the present specification, the organic or inorganic moiety bonded to $Z^{71}$ in the general formula (7) is referred to as a ligand.

$Z^{71}$ is preferably Al, B, V, Ti, Si, Zr, Ge, Sn, Cu, Y, Zn, Ga, Nb, Ta, Bi, P, As, Sc, Hf, or Sb, and more preferably Al, B, or P.

$X^{71}$ represents O, S, an alkylene group having 1 to 10 carbon atoms, a halogenated alkylene group having 1 to 10 carbon atoms, an arylene group having 6 to 20 carbon atoms, or a halogenated arylene group having 6 to 20 carbon atoms. These alkylene groups and arylene groups optionally have a substituent or a heteroatom in their structure.

Specifically, they may have a halogen atom, a chain or cyclic alkyl group, an aryl group, an alkenyl group, an alkoxy group, an aryloxy group, a sulfonyl group, an amino group, a cyano group, a carbonyl group, an acyl group, an amide group, or a hydroxy group as a substituent instead of hydrogen on the alkylene groups and arylene groups, or they may have a structure in which nitrogen, sulfur, or oxygen is introduced instead of a carbon on the alkylene groups and arylene groups. When n72 is 1 and n73 is 2 to 4, n73 $X^{71}$ (2 to 4 $X^{71}$) are optionally bonded to each other. Such an example may include a ligand such as ethylenediaminetetraacetic acid.

$L^{71}$ represents a halogen atom, a cyano group, an alkyl group having 1 to 10 carbon atoms, a halogenated alkyl group having 1 to 10 carbon atoms, an aryl group having 6 to 20 carbon atoms, a halogenated aryl group having 6 to 20 carbon atoms, or $-Z^{73}Y^{73}$ ($Z^{73}$ and $Y^{73}$ will be described later) . The alkyl groups and aryl groups here, like $X^{71}$, also optionally have a substituent or a heteroatom in their structure, and when n71 is 2 to 8, n71 $L^{71}$ (2 to 8 $L^{71}$) are optionally bonded to each other to form a ring. $L^{71}$ is preferably a fluorine atom or a cyano group.

$Y^{71}$, $Y^{72}$, and $Z^{73}$ each independently represent O, S, $NY^{74}$, a hydrocarbon group, or a fluorinated hydrocarbon group. $Y^{71}$ and $Y^{72}$ are preferably O, S, or $NY^{74}$, and more preferably O. The oxalato complex compound (7) is characterized by that there are bonds to $Z^{71}$ by $Y^{71}$ and $Y^{72}$ in the same ligand, so that these ligands constitute a chelate structure with $Z^{71}$. This chelate effect improves the heat resistance, chemical stability, and hydrolysis resistance of this compound. Although the constant n72 in this ligand is 0 or 1, in the case of 0, this chelate ring becomes a 5-membered ring and thus the chelate effect is most strongly exerted and stability is increased, which is particularly preferred.

Note that, in the present specification, the fluorinated hydrocarbon group is a group in which at least one hydrogen atom of a hydrocarbon group is replaced by a fluorine atom.

$Y^{73}$ and $Y^{74}$ are each independently H, F, an alkyl group having 1 to 10 carbon atoms, a halogenated alkyl group having 1 to 10 carbon atoms, an aryl group having 6 to 20 carbon atoms, or a halogenated aryl group having 6 to 20 carbon atoms, and these alkyl groups and aryl groups optionally have a substituent or a heteroatom in their structure. Also, in the case where there is a plurality of $Y^{73}$ or $Y^{74}$, they are optionally bonded to each other to form a ring.

Also, the constant n73 related to the number of ligands mentioned above is an integer of 1 to 4, preferably 1 or 2, and more preferably 2. In addition, the constant n71 related to the number of ligands mentioned above is an integer of 0 to 8, preferably an integer of 0 to 4, and more preferably 0, 2, or 4. Furthermore, it is preferable that n71 is 2 when n73 is 1 and n71 is 0 when n73 is 2.

In the general formula (7), the alkyl group, the halogenated alkyl group, the aryl group, and the halogenated aryl group include those having another functional group such as a branch, a hydroxy group, or an ether bond.

It is preferable that the oxalato complex compound (7) is a compound represented by the general formula (7-1):

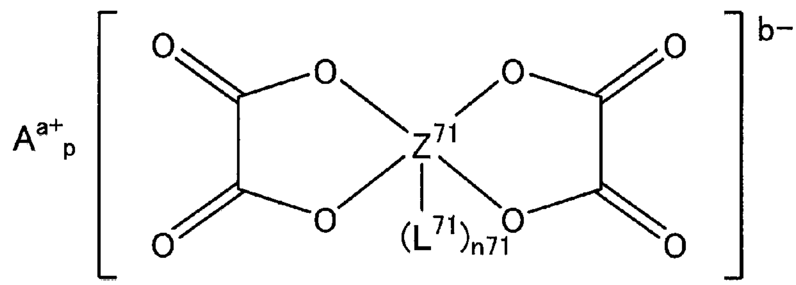

(7-1)

wherein $A^{a+}$, a, b, p, n71, $Z^{71}$, and $L^{71}$ are as mentioned above, or
a compound represented by the general formula (7-2):

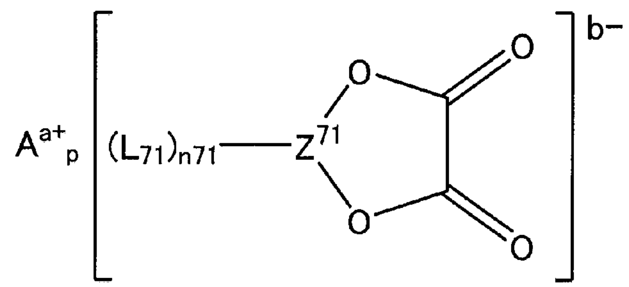

(7-2)

wherein $A^{a+}$, a, b, p, n71, $Z^{71}$, and $L^{71}$ are as mentioned above.

Examples of the oxalato complex compound (7) include a lithium oxalato borate salt, and include

lithium bis(oxalato)borate (LIBOB) represented by the following formula:

LIBOB

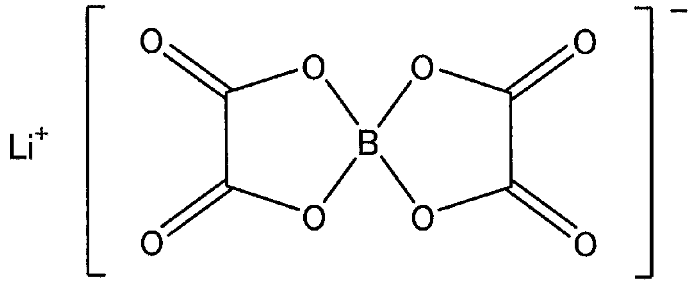

and

lithium difluorooxalatoborate (LIDFOB) represented by the following formula:

LIDFOB

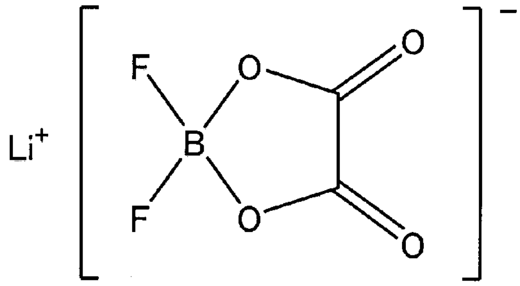

Examples of the oxalato complex compound (7) also include

lithium difluorooxalatophosphanite (LIDFOP) represented by the following formula:

LIDFOP

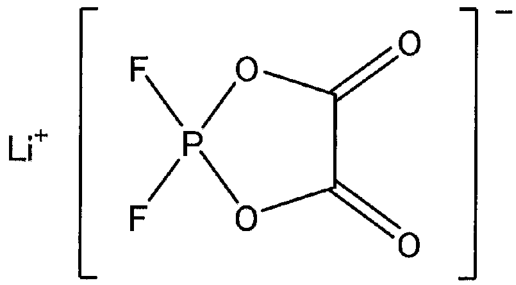

lithium tetrafluorooxalatophosphanite (LITFOP) represented by the following formula:

LITFOP

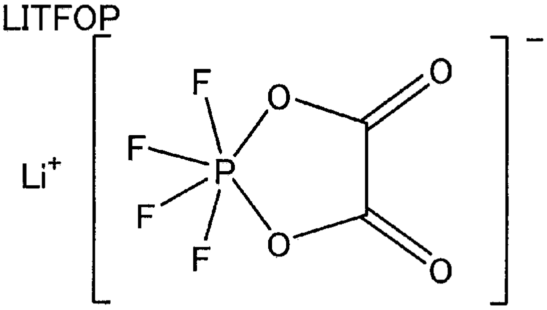

and

lithium bis(oxalato)difluorophosphanite represented by the following formula:

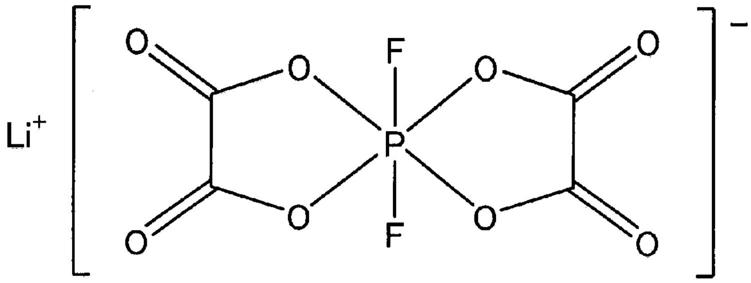

Besides, specific examples of dicarboxylic acid complex salts in which the complex center element is boron include lithium bis(malonato)borate, lithium difluoro(malonato)borate, lithium bis(methylmalonato)borate, lithium difluoro(methylmalonato)borate, lithium bis(dimethylmalonato)borate, and lithium difluoro(dimethylmalonato)borate.

Specific examples of dicarboxylic acid complex salts in which the complex center element is phosphorus include lithium tris(oxalato)phosphate, lithium tris(malonato)phosphate, lithium difluorobis(malonato)phosphate, lithium tetrafluoro(malonato)phosphate, lithium tris(methylmalonato)phosphate, lithium difluorobis(methylmalonato)phosphate, lithium tetrafluoro(methylmalonato)phosphate, lithium tris(dimethylmalonato)phosphate, lithium difluorobis(dimethylmalonato)phosphate, and lithium tetrafluoro(dimethylmalonato)phosphate.

Specific examples of dicarboxylic acid complex salts in which the complex center element is aluminum include $LiAl(C_2O_4)_2$ and $LiAlF_2(C_2O_4)$.

In particular, since an increase in the viscosity of the positive electrode mixture can be further suppressed, lithium bis(oxalato)borate, lithium difluoro(oxalato)borate, lithium tris(oxalato)phosphate, lithium difluorobis(oxalato)phosphate, and lithium tetrafluoro(oxalato)phosphate are more suitably used.

As the oxalato complex compound (7), lithium bis(oxalato)borate is particularly preferred.

In the positive electrode mixture of the present disclosure, the content of the additive (D) is preferably 0.001 to 5% by mass, more preferably 0.005% by mass or more, and still more preferably 0.01% by mass or more, and it is more preferably 3.0% by mass or less, still more preferably 2.5% by mass or less, and particularly preferably 2.0% by mass or less, based on the total mass of the PVdF (A), and the positive electrode active material (C). When the content of the additive (D) is in the range described above, an increase in the viscosity of the positive electrode mixture can be further suppressed, and batteries having an even higher residual capacity rate and an even higher recovery capacity rate after high-temperature storage can be obtained.

Organic solvent

It is preferable that the positive electrode mixture of the present disclosure contains an organic solvent. Examples of the organic solvent described above may include a low-boiling point general-purpose organic solvent, such as: a nitrogen-containing organic solvent such as N-methyl-2-pyrrolidone, N,N-dimethylacetamide, and dimethylformamide; a ketone solvent such as acetone, methyl ethyl ketone, cyclohexanone, and methyl isobutyl ketone; an ester solvent such as ethyl acetate and butyl acetate; an ether solvent such as tetrahydrofuran and dioxane; as well as a mixed solvent thereof. Among these, it is preferable that the organic solvent described above is N-methyl-2-pyrrolidone and/or N,N-dimethylacetamide from the viewpoint of excellent applicability.

In the positive electrode mixture of the present disclosure, the content of the organic solvent described above is determined in consideration of the applicability to the current collector, thin film formability after drying, and other factors. In the positive electrode mixture of the present disclosure, the total content of the PVdF (A), and the positive electrode active material (C) is preferably 50 to 90% by mass, more preferably 60 to 85% by mass, and still more preferably 65 to 80% by mass. Also, in the positive electrode mixture of the present disclosure, the total content of the additive (C) and the organic solvent is preferably 10 to 50% by mass, more preferably 15 to 40% by mass, and still more preferably 20 to 35% by mass.

In addition, it is desirable that the PVdF (A) are used in a small particle size, with an average particle size of 1,000 μm or less, in particular 350 μm or less, in order to enable rapid dissolution in the organic solvent described above.

Conductive agent

It is preferable that the positive electrode mixture of the present disclosure contains a conductive agent. Examples of the conductive agent described above include a carbon material such as a carbon black including acetylene black and Ketjen black, and graphite, a carbon fiber, a carbon nanotube, and a carbon nanohorn.

In the positive electrode mixture of the present disclosure, the content ratio of the PVdF (A) to the conductive

agent is 5/95 to 90/10 in mass ratio.

Examples of the method for preparing the positive electrode mixture of the present disclosure include a method in which, in a solution of the PVdF (A) dissolved in an organic solvent, the positive electrode active material (C), the additive (D), and if desired, the conductive agent are dispersed and mixed. Alternatively, the positive electrode mixture may be prepared by a method in which the PVdF (A) are first mixed with the positive electrode active material (C), and then the organic solvent, the additive (D), and if desired, the conductive agent are added, or the positive electrode mixture may be prepared by a method in which, to a solution of the PVdF (A) dissolved in an organic solvent, the additive (D), and if desired, the conductive agent are added, and after mixing, the positive electrode active material (C) is further added and mixed.

The viscosity of the positive electrode mixture of the present disclosure is, since application is easy and it is also easy to obtain a positive electrode mixture layer having the desired thickness, preferably 1,000 to 80,000 mPa·s, more preferably 3,000 to 70,000 mPa·s, and still more preferably 5,000 to 60,000 mPa·s. The viscosity described above can be measured at 25°C with a B-type viscometer.

## Positive electrode

A positive electrode of the present disclosure is formed from the positive electrode mixture mentioned above. Examples of the method for forming a positive electrode using the positive electrode mixture mentioned above include a method in which the positive electrode mixture mentioned above is applied to a current collector, dried, and pressed to form a thin positive electrode mixture layer on the current collector to provide a thin-film electrode. That is, in one preferred embodiment, the positive electrode of the present disclosure comprises a current collector and a positive electrode mixture layer formed from the positive electrode mixture mentioned above on the current collector described above.

Examples of the current collector described above include a metal foil or a metal mesh, such as iron, stainless steel, copper, aluminum, nickel, and titanium, and among these, an aluminum foil is preferred.

## Secondary battery

A secondary battery of the present disclosure comprises the positive electrode mentioned above. It is preferable that the secondary battery of the present disclosure further comprises, in addition to the positive electrode mentioned above, a negative electrode and a non-aqueous electrolyte.

Although the non-aqueous electrolyte described above is not limited, one or two or more of the following known solvents can be used: propylene carbonate, ethylene carbonate, butylene carbonate, γ-butyrolactone, 1,2-dimethoxyethane, 1,2-diethoxyethane, dimethyl carbonate, diethyl carbonate, and ethyl methyl carbonate. For the electrolyte, any of those conventionally known can be used, and $LiClO_4$, $LiAsF_6$, $LiPF_6$, $LiBF_4$, LiCl, LiBr, $CH_3SO_3Li$, $CF_3SO_3Li$, cesium carbonate, and others can be used.

The positive electrode mixture of the present disclosure is useful for non-aqueous electrolyte secondary batteries, not only for lithium-ion secondary batteries using the liquid electrolyte described above, but also for polymer electrolyte lithium secondary batteries. It is also useful for electric double layer capacitors.

Although the embodiments have been described above, it will be understood that a wide variety of modifications can be made in the form and details without departing from the spirit and scope of the claims.

<1> According to the first viewpoint of the present disclosure, provided is a positive electrode mixture comprising:

a polyvinylidene fluoride (A);
a positive electrode active material (C) represented by the general formula (C) : $Li_yNi_{1-x}M_xO_2$,

wherein x is $0.01 \leq x \leq 0.7$, y is $0.9 \leq y \leq 2.0$, and M represents a metal atom excluding Li and Ni; and

an additive (D),
wherein the polyvinylidene fluoride (A) is a combination of:
(A2) a polymer containing vinylidene fluoride unit and a fluorinated monomer unit excluding vinylidene fluoride unit and tetrafluoroethylene unit; and
at least one selected from the group consisting of (A3) a polymer containing vinylidene fluoride unit and a polar group-containing monomer unit, and (A4) a polymer containing vinylidene fluoride unit, a fluorinated monomer unit excluding vinylidene fluoride unit and tetrafluoroethylene unit, and a polar group-containing monomer unit, and
wherein the additive (D) is at least one selected from the group consisting of:

a diketone compound (1) represented by the general formula (1);
an amine compound (2) represented by the general formula (2);
an organophosphorus compound (3);
a sulfur-containing compound (4);
an aminocarbonate compound (5) represented by the general formula (5) ;
a carboxylic acid (6) excluding the aminocarbonate compound (5); and
an oxalato complex compound (7):

the general formula (1):

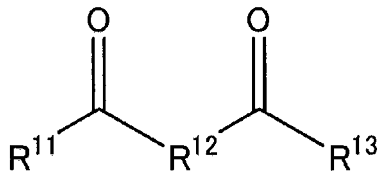

(1)

wherein $R^{11}$ and $R^{13}$ independently represent a monovalent hydrocarbon group optionally containing a heteroatom or a carbonyl group, $R^{12}$ represents a divalent hydrocarbon group or a divalent heteroatom, $R^{11}$, $R^{12}$, and $R^{13}$ are optionally bonded to each other to form a ring;
the general formula (2):

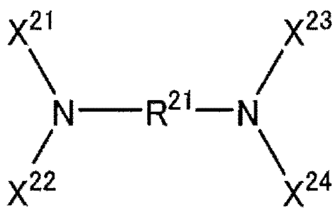

(2)

wherein $R^{21}$ represents a single bond or a divalent organic group, $X^{21}$ to $X^{24}$ independently represent a hydrogen atom, a substituted or unsubstituted alkyl group, or an aryl group optionally bonded to a nitrogen atom via a sulfonyl group, $X^{21}$ and $X^{23}$ are optionally bonded to each other to form a ring, and $X^{22}$ and $X^{24}$ are optionally bonded to each other to form a ring; and
the general formula (5):

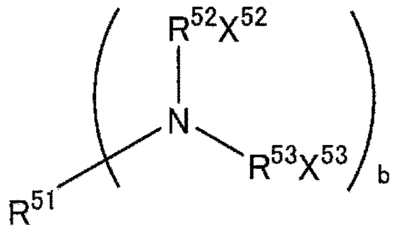

(5)

wherein $R^{51}$ represents an organic group, $R^{52}$ represents a single bond or an alkylene group, $X^{52}$ represents -OH or $-COOZ^{52}$, where $Z^{52}$ is a hydrogen atom or an alkali metal, $R^{53}$ represents a single bond or an alkylene group, $X^{53}$ is -H, -OH or $-COOZ^{53}$, where $Z^{53}$ is a hydrogen atom or an alkali metal, and b represents 1 or 2.

<2> According to the second viewpoint of the present disclosure,
provided is the positive electrode mixture according to the first viewpoint, wherein the fluorinated monomer unit contained in the polyvinylidene fluoride (A2) and the polyvinylidene fluoride (A4) is at least one selected from the group consisting of hexafluoropropylene unit, a fluoroalkyl vinyl ether unit, chlorotrifluoroethylene unit, and 2,3,3,3-tetrafluoropropene unit.
<3> According to the third viewpoint of the present disclosure,
provided is the positive electrode mixture according to the first or second viewpoint, wherein a mass ratio between the polyvinylidene fluoride (A2) and at least one selected from the group consisting of the polyvinylidene fluoride

(A3) and the polyvinylidene fluoride (A4) is 95/5 to 5/95.

<4> According to the fourth viewpoint of the present disclosure,

provided is the positive electrode mixture according to any of the first to the third viewpoints, wherein a content of the additive (D) is 0.001 to 5% by mass based on a total mass of the polyvinylidene fluoride (A) and the positive electrode active material (C).

<5> According to the fifth viewpoint of the present disclosure,

provided is the positive electrode mixture according to any of the first to the fourth viewpoints, further comprising an organic solvent.

<6> According to the sixth viewpoint of the present disclosure,

provided is a positive electrode formed from the positive electrode mixture according to any of the first to the fifth viewpoints.

<7> According to the seventh viewpoint of the present disclosure,

provided is a secondary battery comprising the positive electrode according to the sixth viewpoint.

## EXAMPLES

Hereinafter, embodiments of the present disclosure will be described with reference to Examples, but the present disclosure is not limited solely to such Examples.

Each numerical value in Examples was measured according to the following method.

<Compositional features of PVdF (A-I)>

The content of acrylic acid unit in the PVdF (A-I) was measured by acid-base titration of the carboxylic acid group. Specifically, about 0.5 g of the PVdF (A-I) was dissolved in acetone at a temperature of 70 to 80°C. 5 ml of water was added dropwise with vigorous stirring to avoid coagulation of the PVdF (A-I). Titration with aqueous NaOH with a concentration of 0.1N was performed until complete neutralization of acidity at a neutral transition at about -270 mV. Based on the measured acid equivalent, the amount of substance of acrylic acid contained in 1 g of the PVdF (A-I) was determined from the measurement results, and the content of acrylic acid unit was calculated.

<Compositional features of PVdF (A-III)>

The ratio of VdF and HFP was measured in the state of polymer solution in LMF-$d_7$ by $^{19}$F-NMR measurement using an NMR analyzer (manufactured by Agilent Technologies, Inc., VNS400MHz).

The areas of the following peaks (A, B, and C) were determined by $^{19}$F-NMR measurement, and the ratio of VdF to HFP was calculated.

A: Area of the peak of -69 ppm to -78 ppm
B: Area of the peak of -86 ppm to -98 ppm
C: Area of the peak of -101 ppm to -122 ppm
Proportion of VdF: $(3B + 3C - 2A)/(3B + 3C) \times 100$ [mol%]
Proportion of HFP: $(2A)/(3B + 3C) \times 100$ [mol%]

<Weight average molecular weight>

The weight average molecular weight was measured by gel permeation chromatography (GPC). The weight average molecular weight was calculated from data measured with dimethylformamide (DMF) as the solvent at a flow rate of 1.0 ml/min using AS-8010, CO-8020, and columns (three GMHHR-H connected in series) manufactured by Tosoh Corporation, and RID-10A manufactured by SHIMADZU CORPORATION (reference: polystyrene).

<Melting point>

The melting point was determined as the temperature corresponding to the local maximum value in the heat-of-fusion curve when the temperature was increased at a rate of 10°C/min using a differential scanning calorimetry (DSC) apparatus.

<Viscosity>

As for the viscosity of the positive electrode mixture, measurement was performed using a B-type viscometer

(manufactured by Toki Sangyo Co., Ltd., TV-10M) under the conditions of 25°C, rotor No. M4, and a rotation speed of 6 rpm, and the measured value after 10 minutes had passed from the start of measurement was defined as the viscosity.

<Rate of change in viscosity>

The viscosity at the time of mixture preparation ($\eta 0$) and the viscosity after n hours from the mixture preparation ($\eta n$) were each measured, and the rate of change in viscosity (Xn) was determined by the following expression.

$$Xn = \eta n/\eta 0 \times 100\ [\%]$$

The use of a positive electrode mixture with a rate of change in viscosity (X) of 200% or less enables preparation of a positive electrode that exhibits good characteristics. When a positive electrode mixture with a rate of change in viscosity (X) of greater than 200% and 300% or less is used, disadvantages occur, such as the inability to form a positive electrode mixture layer having a smooth surface. A positive electrode mixture with a rate of change in viscosity (X) of greater than 300% is difficult to apply.

(Fabrication of lithium-ion secondary battery)

The positive electrode comprising the positive electrode mixture layer on one side, prepared in Examples and Comparative Examples, was cut into a shape having a coated section (positive electrode mixture layer) with a width of 50 mm and a length of 30 mm, and an uncoated section with a width of 5 mm and a length of 9 mm.

To 98 parts by mass of artificial graphite, 1 part by mass of an aqueous dispersion of carboxymethyl cellulose sodium (carboxymethyl cellulose sodium concentration: 1% by mass) and 1 part by mass of an aqueous dispersion of a styrene butadiene rubber (styrene butadiene rubber concentration: 50% by mass) were added as the thickening agent and the binder, and the mixture was mixed with a disperser to make it slurried. The obtained slurry was applied to a copper foil with a thickness of 20 μm, which was dried, rolled with a pressing machine, and cut into a shape having a coated section (negative electrode material layer) with a width of 52 mm and a length of 32 mm, and an uncoated section with a width of 5 mm and a length of 9 mm to provide a negative electrode.

(Fabrication of aluminum laminate cell)

The positive electrode and negative electrode described above were placed facing each other via a microporous polyethylene film (separator) with a thickness of 20 μm, the non-aqueous electrolyte obtained as described above was injected, and after the non-aqueous electrolyte described above sufficiently penetrated the separator and other components, the cell was sealed and aged. Thereafter, charging was performed at 25°C to 4.2 V (or 4.3 V) at a constant current corresponding to 0.2 C, and then discharging was performed to 3.0 V at a constant current of 0.2 C. This was carried out for two cycles to stabilize the battery to fabricate a lithium-ion secondary battery (aluminum laminate cell).

(Measurement of battery characteristics of lithium-ion secondary battery using NMC811)

For the obtained aluminum laminate cell, a 60°C storage test was carried out as described below to examine the residual capacity rate and the recovery capacity rate.

(Measurement of residual capacity rate and recovery capacity rate in 60°C storage test of lithium-ion secondary battery using NMC811)

The secondary battery produced as described above was charged at 25°C to 4.2 V at a constant current of 1 C, and charging was then performed at a constant voltage of 4.2 V until the current value reached 0.05 C. Thereafter, discharging was performed to 3.0 V at a constant current of 1 C to determine the initial discharge capacity (mAh). At 25°C, after charging to 4.2 V at a constant current of 1 C, charging was performed at a constant voltage of 4.2 V until the current value reached 0.05 C. Then, the secondary battery was stored under the condition of 60°C for 30 days. Thereafter, discharging was performed to 3.0 V at a constant current of 1 C under the condition of 25°C to determine the residual capacity (mAh) . Here, the residual capacity (mAh) was divided by the initial discharge capacity (mAh) to determine the residual capacity rate (%).

$$\text{Residual capacity rate (\%)} = \text{Residual capacity (mAh)}/\text{Initial discharge capacity (mAh)} \times 100$$

Also, the battery described above for which the residual capacity had been measured was charged to 4.2 V at a constant current of 1 C under the condition of 25°C, charging was then performed at a constant voltage of 4.2 V until the current value reached 0.05 C, and then discharging was performed to 3.0 V at a constant current of 1 C to determine the recovery capacity (mAh). Here, the recovery capacity (mAh) was divided by the initial discharge capacity (mAh) to determine the recovery capacity rate (%).

$$\text{Recovery capacity rate (\%)} = \text{Recovery capacity (mAh)}/\text{Initial discharge capacity (mAh)} \times 100$$

(Measurement of battery characteristics of lithium-ion secondary battery using NCA)

For the obtained aluminum laminate cell, an 85°C storage test was carried out as described below to examine the residual capacity rate and the recovery capacity rate.

(Measurement of residual capacity rate and recovery capacity rate in 85°C storage test of lithium-ion secondary battery using NCA)

The secondary battery produced as described above was charged at 25°C to 4.3 V at a constant current of 1 C, and charging was then performed at a constant voltage of 4.3 V until the current value reached 0.05 C. Thereafter, discharging was performed to 3.0 V at a constant current of 1 C to determine the initial discharge capacity (mAh). At 25°C, after charging to 4.3 V at a constant current of 1 C, charging was performed at a constant voltage of 4.3 V until the current value reached 0.05 C. Then, the secondary battery was stored under the condition of 85°C for 30 days. Thereafter, discharging was performed to 3.0 V at a constant current of 1 C under the condition of 25°C to determine the residual capacity (mAh) . Here, the residual capacity (mAh) was divided by the initial discharge capacity (mAh) to determine the residual capacity rate (%).

$$\text{Residual capacity rate (\%)} = \text{Residual capacity (mAh)}/\text{Initial discharge capacity (mAh)} \times 100$$

Also, the battery described above for which the residual capacity had been measured was charged to 4.3 V at a constant current of 1 C under the condition of 25°C, charging was then performed at a constant voltage of 4.3 V until the current value reached 0.05 C, and then discharging was performed to 3.0 V at a constant current of 1 C to determine the recovery capacity (mAh). Here, the recovery capacity (mAh) was divided by the initial discharge capacity (mAh) to determine the recovery capacity rate (%).

$$\text{Recovery capacity rate (\%)} = \text{Recovery capacity (mAh)}/\text{Initial discharge capacity (mAh)} \times 100$$

In Examples and Comparative Examples, the following polymers were used.

<PVdF (A)>

A-I: PVdF containing acrylic acid unit

content of acrylic acid unit 1.0 mol%
weight average molecular weight 1,100,000

melting point 165°C

A-III: PVdF containing HFP unit

VdF/HFP = 94.5/5.5 (mol%)
weight average molecular weight 600,000
melting point 132°C

Example 1

The PVdF (A-I) and the PVdF (A-III) were each dissolved in N-methyl-2-pyrrolidone (NMP) to prepare a PVdF (A-I) solution and a PVdF (A-III) solution with a concentration of 8% by mass. 20 g of the obtained PVdF (A-I) solution, 5 g of the PVdF (A-III) solution, 0.05 g of acetylacetone as the additive (D), and 2 g of acetylene black as the conductive agent were mixed using a stirrer to obtain a mixed solution. To the obtained mixed solution, 96 g of the positive electrode active material (C) ($LiNi_{0.8}Mn_{0.1}Co_{0.1}O_2$ (NMC811)) was added, NMP was further added such that the solid concentration was 71% by mass, and the mixture was mixed using a stirrer to obtain a positive electrode mixture. The entire amount of the obtained positive electrode mixture was transferred to a polyethylene bottle, and the viscosity was measured by the method described above. For the obtained positive electrode mixture, the viscosity was measured after each specified time and the rate of change in viscosity was calculated. The prepared positive electrode mixture was uniformly applied to an electrode current collector composed of an aluminum foil with a width of 10 cm, a length of 45 cm, and a thickness of 20 μm, and NMP was completely volatilized to fabricate a positive electrode. Using the obtained positive electrode, a lithium-ion secondary battery was fabricated by the method described above, and the residual capacity rate and recovery capacity rate in the 60°C storage test were evaluated. The results are shown in Table 1.

Examples 2 to 8 and Comparative Examples 1 to 2

Positive electrode mixtures, positive electrodes, and lithium-ion secondary batteries were obtained in the same manner as in Example 1, except that the types and amounts added of the PVdF (A), and the additive (D) were changed to those described in Table 1. The obtained positive electrode mixtures and lithium-ion secondary batteries were evaluated by the methods described above. The results are shown in Table 1.

[Table 1]

Table 1

| | | | Example 1 | Example 2 | Example 3 | Example 4 | Example 5 | Example 6 | Example 7 | Example 8 | Comparative Example 1 | Comparative Example 2 |
|---|---|---|---|---|---|---|---|---|---|---|---|---|
| PVdF(A) | A-I | (g) | 1.6 | 1.4 | 1.5 | 1.0 | 1.2 | 1.8 | 1.5 | 0.2 | 1.6 | 1.0 |
| | A-III | (g) | 0.4 | 0.6 | 0.5 | 1.0 | 0.8 | 0.2 | 0.5 | 1.8 | 0.4 | 1.0 |
| Positive electrode active material (C) | NMC811 | (g) | 96.0 | 96.0 | 96.0 | 96.0 | 96.0 | 96.0 | 96.0 | 96.0 | 96.0 | 96.0 |
| Additive (D) | Acetylacetone | (g) | 0.05 | | | | | | | 0.05 | | |
| | 1,3-Propanesultone | (g) | | 0.05 | | | | | | | | |
| | Ethylenediamine | (g) | | | 0.05 | | | | | | | |
| | Triphenylphosphine | (g) | | | | 0.5 | | | | | | |
| | Nitritotriacetic acid | (g) | | | | | 0.05 | | | | | |
| | Tartaric acid | (g) | | | | | | 0.05 | | | | |
| | LiBOB | (g) | | | | | | | 0.5 | | | |
| Conductive agent | Acetylene black | (g) | 2.0 | 2.0 | 2.0 | 2.0 | 2.0 | 2.0 | 2.0 | 2.0 | 2.0 | 2.0 |
| Viscosity at the time of mixture preparation | η0 | (mPa·s) | 24900 | 22100 | 23100 | 21300 | 19700 | 26300 | 24200 | 18200 | 27900 | 22200 |
| Rate of change in viscosity | X24 | (%) | 97 | 101 | 132 | 108 | 102 | 101 | 114 | 95 | 278 | >300 |
| | X96 | (%) | 103 | 105 | 141 | 131 | 104 | 104 | 128 | 100 | >300 | >300 |
| | X168 | (%) | 114 | 119 | 148 | 156 | 120 | 115 | 146 | 113 | >300 | >300 |
| 60°C Storage test | Residual capacity rate (%) | | 93.6 | 90.1 | 89.5 | 88.8 | 89.1 | 9.4 | 8.4 | 93.8 | 79.6 | 77.9 |
| | Recovery capacity rate (%) | | 93.1 | 95.9 | 92.7 | 96.2 | 95.8 | 93.1 | 93.0 | 92.5 | E4.4 | 82.1 |

Examples 9 to 16 and Comparative Examples 3 to 4

Positive electrode mixtures, positive electrodes, and lithium-ion secondary batteries were obtained in the same manner as in Example 1, except that the types and amounts added of the PVdF (A), and the additive (D) were changed to those described in Table 2 and that the positive electrode active material (C) was changed to $LiNi_{0.82}Co_{0.15}Al_{0.03}O_2$ (NCA) . The obtained positive electrode mixtures and lithium-ion secondary batteries were evaluated by the methods described above. The results are shown in Table 2.

[Table 2]

Table 2

| | | | Example 9 | Example 10 | Example 1.9 | Example 12 | Example 13 | Example 14 | Example 15 | Example 16 | Comparative Example 3 | Comparative Example 4 |
|---|---|---|---|---|---|---|---|---|---|---|---|---|
| PVdF(A) | A-I | (g) | 1.6 | 1.4 | 1.5 | 1.0 | 1.2 | 1.8 | 1.5 | 0.2 | 1.6 | 1.0 |
| | A-III | (g) | 0.4 | 0.6 | 0.5 | 1.0 | 0.8 | 0.2 | 0.5 | 1.8 | 0.4 | 1.0 |
| Positive electrode active material (C) | NCA | (g) | 96.0 | 96.0 | 96.0 | 96.0 | 96.0 | 96.0 | 96.0 | 96.0 | 96.0 | 96.0 |
| Additive (D) | Acetylacetone | (g) | 0.5 | | | | | | | 1.0 | | |
| | 1,3-Propanesultone | (g) | | 0.5 | | | | | | | | |
| | Ethylenediamine | (g) | | | 1.0 | | | | | | | |
| | Triphenylphosphine | (g) | | | | 1.0 | | | | | | |
| | Nitrilotriacetic acid | (g) | | | | | 0.5 | | | | | |
| | Tartaric acid | (g) | | | | | | 0.5 | | | | |
| | LiBOB | (g) | | | | | | | 1.0 | | | |
| Conductive agent | Acetylene black | (g) | 2.0 | 2.0 | 2.0 | 2.0 | 2.0 | 2.0 | 2.0 | 2.0 | 2.0 | 2.0 |
| Viscosity at the time of mixture preparation | η0 | (mPa.s) | 24900 | 22100 | 23100 | 21300 | 19700 | 26300 | 24200 | 18200 | 27900 | 22200 |
| Rate of change in viscosity | X24 | (%) | 97 | 101 | 132 | 108 | 102 | 102 | 103 | 95 | >300 | >300 |
| | X96 | (%) | 103 | 105 | 141 | 152 | 104 | 108 | 133 | 100 | >300 | >300 |
| | X168 | (%) | 114 | 119 | 165 | 187 | 117 | 114 | 191 | 113 | >300 | >300 |
| 85°C Storage test | Residual capacity rate (%) | | 79.3 | 74.3 | 75.1 | 73.3 | 73.9 | 80.1 | 70.0 | 78.4 | 61.2 | 58.3 |
| | Recovery capacity rate (%) | | 77.1 | 80.1 | 76.1 | 75.6 | 81.3 | 75.5 | 75.0 | 74.9 | 63.1 | 59.9 |

## Claims

**1.** A positive electrode mixture comprising:

a polyvinylidene fluoride (A);
a positive electrode active material (C) represented by the general formula (C): $Li_yNi_{1-x}M_xO_2$,

wherein x is $0.01 \leq x \leq 0.7$, y is $0.9 \leq y \leq 2.0$, and M represents a metal atom excluding Li and Ni; and

an additive (D),
wherein the polyvinylidene fluoride (A) is a combination of:
(A2) a polymer containing vinylidene fluoride unit and a fluorinated monomer unit excluding vinylidene fluoride unit and tetrafluoroethylene unit; and
at least one selected from the group consisting of (A3) a polymer containing vinylidene fluoride unit and a polar group-containing monomer unit, and (A4) a polymer containing vinylidene fluoride unit, a fluorinated monomer unit excluding vinylidene fluoride unit and tetrafluoroethylene unit, and a polar group-containing monomer unit, and
wherein the additive (D) is at least one selected from the group consisting of:

a diketone compound (1) represented by the general formula (1);
an amine compound (2) represented by the general formula (2);
an organophosphorus compound (3);
a sulfur-containing compound (4);
an aminocarbonate compound (5) represented by the general formula (5) ;
a carboxylic acid (6), excluding the aminocarbonate compound (5); and
an oxalato complex compound (7):

the general formula (1):

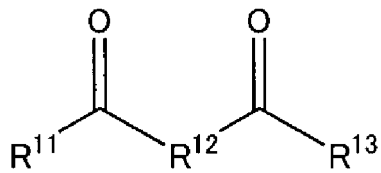

(1)

wherein $R^{11}$ and $R^{13}$ independently represent a monovalent hydrocarbon group optionally containing a heteroatom or a carbonyl group, $R^{12}$ represents a divalent hydrocarbon group or a divalent heteroatom, $R^{11}$, $R^{12}$, and $R^{13}$ are optionally bonded to each other to form a ring;
the general formula (2):

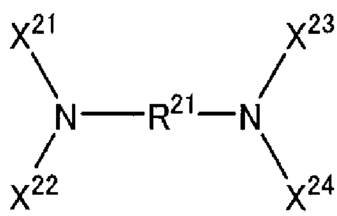

(2)

wherein $R^{21}$ represents a single bond or a divalent organic group, $X^{21}$ to $X^{24}$ independently represent a hydrogen atom, a substituted or unsubstituted alkyl group, or an aryl group optionally bonded to a nitrogen atom via a sulfonyl group, $X^{21}$ and $X^{23}$ are optionally bonded to each other to form a ring, and $X^{22}$ and $X^{24}$ are optionally bonded to each other to form a ring; and
the general formula (5):

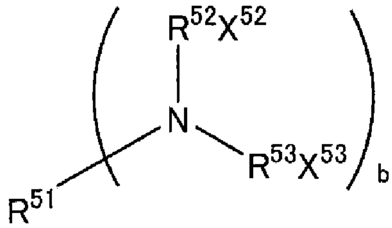

(5)

wherein $R^{51}$ represents an organic group, $R^{52}$ represents a single bond or an alkylene group, $X^{52}$ represents -OH or $-COOZ^{52}$, where $Z^{52}$ is a hydrogen atom or an alkali metal, $R^{53}$ represents a single bond or an alkylene group, $X^{53}$ is -H, -OH or $-COOZ^{53}$, where $Z^{53}$ is a hydrogen atom or an alkali metal, and b represents 1 or 2.

2. The positive electrode mixture according to claim 1, wherein the fluorinated monomer unit contained in the polyvinylidene fluoride (A2) and the polyvinylidene fluoride (A4) is at least one selected from the group consisting of hexafluoropropylene unit, a fluoroalkyl vinyl ether unit, chlorotrifluoroethylene unit, and 2,3,3,3-tetrafluoropropene unit.

3. The positive electrode mixture according to claim 1 or 2, wherein a mass ratio of the polyvinylidene fluoride (A2) to at least one selected from the group consisting of the polyvinylidene fluoride (A3) and the polyvinylidene fluoride (A4) is 95/5 to 5/95.

4. The positive electrode mixture according to any one of claims 1 to 3, wherein a content of the additive (D) is 0.001 to 5% by mass based on a total mass of the polyvinylidene fluoride (A) and the positive electrode active material (C).

5. The positive electrode mixture according to any one of claims 1 to 4, further comprising an organic solvent.

6. A positive electrode formed from the positive electrode mixture according to any one of claims 1 to 5.

7. A secondary battery comprising the positive electrode according to claim 6.

**INTERNATIONAL SEARCH REPORT**

International application No.
**PCT/JP2022/019167**

**A. CLASSIFICATION OF SUBJECT MATTER**

***H01M 4/131***(2010.01)i; ***H01M 4/1391***(2010.01)i; ***H01M 4/525***(2010.01)i; ***H01M 4/62***(2006.01)i
FI: H01M4/1391; H01M4/62 Z; H01M4/525; H01M4/131

According to International Patent Classification (IPC) or to both national classification and IPC

**B. FIELDS SEARCHED**

Minimum documentation searched (classification system followed by classification symbols)

H01M4/131; H01M4/1391; H01M4/525; H01M4/62

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2012-48917 A (SONY CORP.) 08 March 2012 (2012-03-08)<br>claims, examples 2-1 to 2-16 | 1-7<br>1-7 |
| Y | JP 2011-28898 A (TOYOTA JIDOSHA KABUSHIKI KAISHA) 10 February 2011 (2011-02-10)<br>claims, paragraphs [0023], [0036]-[0038] | 1-7 |
| Y | JP 2019-79710 A (TOYOTA JIDOSHA KABUSHIKI KAISHA) 23 May 2019 (2019-05-23)<br>claims, examples | 1-7 |
| Y | WO 2017/158961 A1 (NEC ENERGY DEVICES LTD.) 21 September 2017 (2017-09-21)<br>claims, examples | 1-7 |
| Y | CN 111916693 A (NANCHANG UNIVERSITY) 10 November 2020 (2020-11-10)<br>claims, examples | 1-7 |
| Y | JP 2015-170551 A (KABUSHIKI KAISHA TOYOTA JIDOSHOKKI) 28 September 2015 (2015-09-28)<br>claims, examples | 1-7 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 July 2022** | **19 July 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (January 2015)

## INTERNATIONAL SEARCH REPORT

International application No.
**PCT/JP2022/019167**

### C. DOCUMENTS CONSIDERED TO BE RELEVANT

| Category* | Citation of document, with indication, where appropriate, of the relevant passages | Relevant to claim No. |
|---|---|---|
| Y | JP 2013-175325 A (HITACHI, LTD.) 05 September 2013 (2013-09-05) claims, paragraphs [0056], [0057], example 3 | 1-7 |
| Y | JP 2017-112041 A (TOYOTA JIDOSHA KABUSHIKI KAISHA) 22 June 2017 (2017-06-22) claims, examples, comparative examples 11, 25 | 1-4, 6, 7 |
| A | | 5 |
| Y | JP 2014-32777 A (TOYOTA JIDOSHA KABUSHIKI KAISHA) 20 February 2014 (2014-02-20) claims, examples | 1-7 |
| Y | JP 2014-78449 A (NIPPON SHOKUBAI CO., LTD.) 01 May 2014 (2014-05-01) claims, examples | 1-7 |
| Y | CN 108987752 A (NINGDE CONTEMPORARY AMPEREX TECHNOLOGY CO., LTD.) 11 December 2018 (2018-12-11) claims, examples | 1-7 |
| Y | JP 2016-46188 A (TOYO INK SC HOLDINGS CO., LTD.) 04 April 2016 (2016-04-04) claims, paragraph [0056], examples 1-1 to 1-29 | 1-7 |
| Y | JP 2020-57606 A (DAIKIN INDUSTRIES, LTD.) 09 April 2020 (2020-04-09) claims, paragraphs [0048], [0060], examples | 1-7 |

Form PCT/ISA/210 (second sheet) (January 2015)

**INTERNATIONAL SEARCH REPORT**
**Information on patent family members**

International application No.
**PCT/JP2022/019167**

| Patent document cited in search report | Publication date (day/month/year) | Patent family member(s) | Publication date (day/month/year) |
|---|---|---|---|
| JP 2012-48917 A | 08 March 2012 | (Family: none) | |
| JP 2011-28898 A | 10 February 2011 | (Family: none) | |
| JP 2019-79710 A | 23 May 2019 | (Family: none) | |
| WO 2017/158961 A1 | 21 September 2017 | US 2019/0051891 A1<br>claims, examples<br>EP 3432387 A1<br>CN 108701813 A | |
| CN 111916693 A | 10 November 2020 | (Family: none) | |
| JP 2015-170551 A | 28 September 2015 | (Family: none) | |
| JP 2013-175325 A | 05 September 2013 | US 2013/0219703 A1<br>claims, paragraphs [0058], [0059], example 3<br>EP 2631972 A1<br>KR 10-2013-0097613 A<br>CN 103296280 A | |
| JP 2017-112041 A | 22 June 2017 | US 2017/0179545 A1<br>claims, examples, comp. examples 11, 25<br>EP 3182499 A1<br>CN 106898827 A<br>KR 10-2017-0073490 A | |
| JP 2014-32777 A | 20 February 2014 | (Family: none) | |
| JP 2014-78449 A | 01 May 2014 | (Family: none) | |
| CN 108987752 A | 11 December 2018 | (Family: none) | |
| JP 2016-46188 A | 04 April 2016 | (Family: none) | |
| JP 2020-57606 A | 09 April 2020 | US 2021/0376319 A1<br>claims, paragraphs [0056], [0068], examples<br>WO 2020/071336 A1<br>EP 3863081 A1<br>CN 112703619 A<br>KR 10-2021-0041097 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013176093 A